# EUROPEAN PATENT APPLICATION

(11) **EP 1 210 933 A1**
(43) Date of publication of application: **05.06.2002**
(21) Application number: 99940682.0
(22) Date of filing: 07.09.1999
(51) Int. Cl.: A61K 7/48, A61K 31/13, A61K 31/135, A61K 31/14, A61K 31/145, A61K 31/16, A61K 31/40, A61K 31/685

(54) **SKIN NORMALIZING AGENTS**

(71) Applicant: Kabushiki Kaisha Soken, Kagawa 769-0200 (JP)
(72) Inventor: Tokuyama, Takashi, Ayauta-gun Kagawa 769-0200 (JP); Jo, Megumi, Ayauta-gun Kagawa 769-0200 (JP)
(74) Representative: Desaix, Anne
(86) International application number: JP9904824
(87) International publication number: WO0117499

(57) **Abstract**

The present invention relates to a skin conditioner containing one or more kinds of ingredient selected from the group consisting of an ammonium salt and ions thereof, a compound represented by the formula (1): 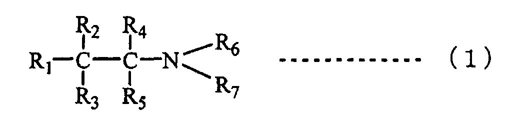 (wherein , the symbols are the same as those defined in the text) and ions thereof and pharmaceutically acceptable salts thereof. Examples of active ingredients of the present invention include L-arginine and ethanolamine. The skin conditioner as claimed in the present invention demonstrates remarkable effectiveness as an agent for restoring the skin's barrier mechanism and function and as an agent for the prevention and treatment of atopic dermatitis.

## Description

### TECHNICAL FIELD

The present invention relates to a skin conditioner that can be used in a broad range of fields including cosmetics, quasi-drugs and pharmaceuticals.

### BACKGROUND ART

In addition to that resulting from aging, human skin and scalp have recently become constantly exposed to risks from external factors such as ultraviolet rays, drying, air-conditioning, air pollution, other irritants and microorganisms, and from internal factors such as contamination by food, water or agricultural chemicals and additives through them, as well as sleep., fatigue and stress.

As a result of these risks, there are many persons with unhealthy skin or persons having skin that at first appears healthy, but is actually in a functionally or structurally unhealthy state. Even persons of an age who ought to inherently have healthy skin have skin that requires the use of cosmetics. However, typical moisture retention agents and oils used in current cosmetics are known to only reach the surface of the skin, and only function as a moisture covering or oil covering without actually acting on the skin.

On the other hand, although oils such as Vaseline have long been used for treatment of symptoms and diseases caused by drying of the skin, these are also merely carried on the surface of the skin, thereby forcing the affected person to wait for the symptoms or disease to heal naturally. In addition, since the effects of typical drugs only act on the particular symptom and do not promote the health of the skin itself, in environments like those found at present, if confronted with the same cause after use is discontinued, there are many cases in which the symptom or disease recurs. In addition, drugs also constantly present the risk of being accompanied by adverse side effects.

### DISCLOSURE OF THE INVENTION

Therefore, the inventors aim to provide a skin conditioner for recovering the skin to its original healthy state in order to restore its beauty and to prevent and recover from diseases of the skin.

The present invention relates a skin conditioner containing one or more kinds of ingredient selected from the group consisting of an ammonium salt and ions thereof, a compound represented by the formula (1): 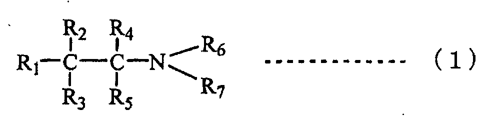 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a carboxyl group, a guanidino group or a lower alkyl group substituted with a guanidino group;
R₄ and R₅ each independently represent a hydrogen atom, a hydroxyl group, a lower alkyl group or an aryl group that may optionally be substituted with a hydroxyl group or an amino group, or a carboxyl group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and ions thereof and pharmaceutically acceptable salts thereof (1).

The present invention also relates to the skin conditioner (1), which is an agent for restoring the skin's barrier mechanism and function (2).

Further, the present invention relates to the skin conditioner (1), which is an agent for the prevention, prevention of exacerbation or treatment of atopic dermatitis (3).

The invention relates the skin conditioners (1) to (3) wherein the compound represented by the formula (1) is selected from the group consisting of L-arginine, ethanolamine, 2-methoxyethylamine, O-phosphorylethanolamine, 2-ethylaminoethanol, diethanolamine, 2-dimethylaminoethanol, choline, 2-amino-2-hydroxymethyl-1,3-propanediol, noradrenalin, phenethylamine, ethylenediamine, taurine, phosphatidylethanolamine, N-(2-hydroxyethyl)acetamide, 2-(methylamino)ethanol, 2-anilinoethanol, 2-(benzylamino)ethanol, 3-amino-1-propanol, 2-amino-1-butanol, putrescine, DL-pyroglutamic acid and triethanolamine (4).

Furthermore, the present invention relates the skin conditioners (1) to (3) containing one or more kinds of ingredient selected from the group consisting of L-arginine and ions thereof and/or pharmaceutically acceptable salts thereof;
an ammonium salt and ions thereof, a compound represented by the following general formula (1)' 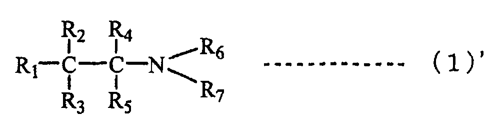 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and ions thereof and pharmaceutically acceptable salts thereof (5).

Moreover, the present invention relates the skin conditioners (1) to (3) containing one or more kinds of ingredient selected from the group consisting of an ammonium salt and/or ions thereof;
a compound represented by the formula (1)': 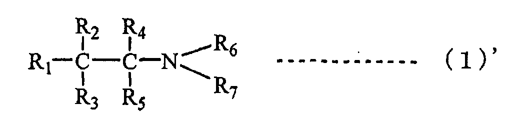 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in. a quaternary form with a lower alkyl group) and ions thereof and pharmaceutically acceptable salts thereof (6).

Still further, the present invention relates the skin conditioners (1) to (3) containing one or more kinds of ingredient selected from the group consisting of L-arginine and ions thereof and/or pharmaceutically acceptable salts thereof;
an ammonium salt and/or ions thereof;
a compound represented by the formula (1)': 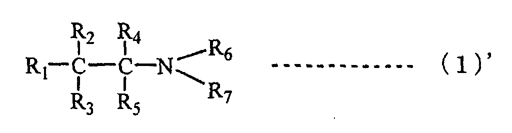 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and ions thereof and pharmaceutically acceptable salts thereof (7).

The invention relates the skin conditioners (5) to (7) wherein the compound represented by the formula (1)' is selected from the group consisting of ethanolamine, 2-methoxyethylamine, O-phosphorylethanolamine, 2-ethylaminoethanol, diethanolamine, 2-dimethylaminoethanol, choline, 2-amino-2-hydroxymethyl-1,3-propanediol, noradrenalin, phenethylamine, ethylenediamine, taurine, phosphatidylethanolamine, N-(2-hydroxyethyl)acetamide, 2-(methylamino)ethanol, 2-anilinoethanol, 2-(benzylamino)ethanol, 3-amino-1-propanol, 2-amino-1-butanol, putrescine, DL-pyroglutamic acid and triethanolamine (8).

Furthermore, the present invention relates to the skin conditioners (1) to (8) containing natural substance preparations (9).

The present invention relates to the skin conditioner (9) wherein the natural substance preparation is a rice preparation (10).

Still further, the present invention relates to the skin conditioners (1) to (10) further containing a moisture retention agent (11).

### BRIEF DESCRIPTION OF THE DRAWINGS

Fig. 1 shows the results of performing a collagen production recovery test on damaged fibroblasts for Examples of the present invention.
Fig. 2 shows the results of a moisture retention duration test on an Example of the present invention.
Fig. 3 shows the results of a moisture retention duration test on an Example of the present invention.
Fig. 4 shows the results of performing a moisture retention ability test on Examples of the present invention.
Fig. 5 shows the results of performing a moisture retention ability test on Examples of the present invention.
Fig. 6 shows the results of a 2-hour moisture retention duration test according to Examples of the present invention.
Fig. 7 shows the results of a 2-hour moisture retention duration test according to Examples of the present invention.
Fig. 8 shows the results of a chapped skin recovery test according to Examples of the present invention.
Fig. 9 shows the overall improvement (usefulness) of using Examples of the present invention in dry eczema, xeroderma and facial dry eczema patients.
Fig. 10 shows improvement of itchiness, sclerosis and cornification by Examples of the present invention.
Fig. 11 shows improvement of scaling and cracking by Examples of the present invention.
Fig. 12 shows improvement of erythema, dryness and wrinkles by Examples of the present invention.
Fig. 13 shows overall improvement (usefulness) of using Examples of the present invention in asteatosis, xeroderma, facial dry eczema and progressive volar keratoderma (keratodermia tylodes palmaris progressiva) patients.
Fig. 14 shows improvement of itchiness, sclerosis and cornification by Examples of the present invention.
Fig. 15 shows improvement of scaling and cracking by Examples of the present invention.
Fig. 16 shows improvement of erythema, dryness and wrinkles by Examples of the present invention.
Fig. 17 shows changes in the severity score of vasodilation by Examples of the present invention.
Fig. 18 shows changes in the severity score of cellular infiltration by Examples of the present invention.
Fig. 19 shows changes in the severity score of keratin hyperplasia by Examples of the present invention.
Fig. 20 shows changes in the severity score of parakeratosis by Examples of the present invention.
Figs. 21 to 31 show the results of a moisture retention duration test performed on atopic skin according to Examples of the present invention.
Figs. 32 to 34 show the results of a moisture retention ability test performed on atopic skin according to Examples of the present invention.
Figs. 35 to 37 show the results of a test of transepidermal moisture evaporation volume performed on atopic skin according to Examples of the present invention.
Fig. 38 shows the results of an allergic reaction inhibition test according to Examples of the present invention.
Figs. 39 to 50 show changes in the severity score performed on atopic skin according to Examples of the present invention.
Fig. 51 shows the results of moisture retention ability tests when applied for a long time on persons with chapped skin according to Example of the present invention.
Fig. 52 shows the results of moisture retention ability tests when applied for a long time on persons with atopic skin according to Example of the present invention.
Figs. 53 to 55 show the results of various skin tests performed according to Examples (especially the one comprising an ammonium ion) of the present invention.

### BEST MODE FOR CARRYING OUT THE INVENTION

First, the definition of each term in the present specification will be described.

The "Lower alkyl group" and the "lower alkyl" are a straight or branched alkyl group having 1 to 10 carbon atoms, and preferably 1 to 5 carbon atoms, examples of which include a methyl group and an ethyl group. The "lower alkoxy group" is the lower alkyl group to which an oxygen atom is attached, examples of which include a methoxy group and an ethoxy group. The "aryl group" is the one having 6 to 18 carbon atoms, and preferably 6 to 10 carbon atoms, examples of which include a phenyl group and α-naphthyl group. The "aralkyl group" is the lower alkyl group to which an aryl group is attached, examples of which include a benzyl group and a phenythyl group. The "substituent" in "that may optionally have a substitutent" is not particularly limited, examples of which include a hydroxyl group, an amino group and a carboxyl group.

The "pharmaceutically acceptable salts" are, for example, pharmaceutically acceptable hydrochloride, hydrobromide, hydrosufate, citrate, acetate, maleate, succinate, methansulfonate and p-toluenesulfonate. The "ammonium salt" means a pharmaceutically acceptable ammonium salt, examples of which include hydrochloride, hydrobromide, hydrosufate, citrate, acetate, maleate, succinate, methansulfonate and p-toluenesulfonate.

The "natural substance preparation" is one obtained using natural substances (for example, bio-components and plant components) as row materials. Examples include pressed natural substances, extracts of natural substances by acid or alkali, hydrated natural substances, extracts of natural substances by organic solvents, oxygen-reacted and fermented natural substances.

"Skin conditioning" essentially means conditioning the epidermis, and the concept may embrace the conditioning of the dermis. "Restoring the skin's barrier mechanism and function" is a concept embraced in the restoration of the epidermis and means restoring the corneal layer of the epidermis (stratum corneum epidermidis) and epidermal keratocytes, promoting the production of a healthy corneal layer of the epidermis and/or normalizing cell differentiation. "Atopic dermatitis" is atopic dermatitis caused by allergic factors and/or damage of the skin's barrier mechanism and functions. "Conditioning" means restoring the skin to its original healthy state.

Next, the skin conditioner according to the present invention will be described.

As to active ingredients of the skin conditioner according to the present invention, there are three types: (i) one or more kinds of ammonium salt (including ions thereof), (ii) the combination of one or more kinds of ammonium salt with one or more kinds of compound of the formula (1) (including salts and ions thereof), and (iii) one or more kinds of compound of the formula (1).

Among the compounds of the formula (1), preferable compound of the formula (1) is the compound wherein R₁, R₂ and R₃ each independently represent a hydrogen atom, a hydroxyl group, an aryl group that may optionally be substituted with a hydroxyl group, an amino group, a lower alkyl group that may optionally be substituted with a hydroxyl group or an amino group, a carboxyl group, a guanidino group or a lower alkyl group substituted with a guanidino group; R₄ and R₅ each independently represent a hydrogen atom, a lower alkyl group that may optionally be substituted with a hydroxyl group or an amino group, or a carboxyl group, or R₄ and R₅, together with an adjacent carbon atom, form a carbonyl group; R₆ and R₇ each independently represent a hydrogen atom, a lower alkyl group that may optionally be substituted with a hydroxyl group, a lower alkylcarbonyl group or an aryl group, or R₆ and R₂ represent alkylene groups, which may optionally have a substituent, that together form a 5-membered ring with an adjacent atom; and, nitrogen atoms in the formula may be in a quaternary form with a lower alkyl group.

More preferable compound of the formula (1) is the compound wherein R₁, R₂ and R₃ each independently represent a hydrogen atom, a hydroxyl group, an aryl group that may optionally be substituted with a hydroxyl group, an amino group, a lower alkyl group that may optionally be substituted with a hydroxyl group or an amino group, a carboxyl group, a guanidino group or a lower alkyl group substituted with a guanidino group; R₄ represents a carboxyl group or R₄ and R₅, together with an adjacent carbon atom, form a carbonyl group; R₅ represents a hydrogen atom, a carboxyl group or a lower alkyl group that may optionally be substituted with a hydroxyl group or an amino group; R₆ and R₇ each independently represent a hydrogen atom, a lower alkyl group that may optionally be substituted with a hydroxyl group, a lower alkylcarbonyl group or an aryl group, or R₆ and R₂ represent alkylene groups, which may optionally have a substituent, that together form a 5-membered ring with an adjacent atom; and, nitrogen atoms in the formula may be in a quaternary form with a lower alkyl group.

Further preferable compound of the formula (1) is the compound wherein R₁, R₂ and R₃ each independently represent a hydrogen atom, an aryl group that may optionally be substituted with a hydroxyl group, a lower alkyl group that may optionally be substituted with a hydroxyl group, a guanidino group or a lower alkyl group substituted with a guanidino group; R₄ represents a carboxyl group; R₅ represents a hydrogen atom, a carboxyl group or a lower alkyl group that may optionally be substituted with a hydroxyl group; R₆ and R₇ each independently represent a hydrogen atom, a lower alkyl group that may optionally be substituted with a hydroxyl group; and, nitrogen atoms in the formula may be in a quaternary form with a lower alkyl group.

Furthermore preferable compound of the formula (1) is the compound wherein R₁ represents a hydroxyl group, an alkyl group, a guanidino group or a lower alkyl group substituted with a guanidino group; R₂ and R₃ each independently represent a hydrogen atom, a hydroxyl group or a lower alkyl group or an aryl group that may optionally be substituted with a hydroxyl group; R₄ represents a carboxyl group; R₅ represents a hydrogen atom, a carboxyl group or a lower alkyl group that may optionally be substituted with a hydroxyl group; R₆ and R₇ each independently represent a hydrogen atom or a lower alkyl group that may optionally be substituted with a hydroxyl group; and, nitrogen atoms in the formula may be in a quaternary form with a lower alkyl group.

The most preferable compound of the formula (1) is the compound wherein R₁ represents a guanidino group or a lower alkyl group substituted with a guanidino group; R₂ and R₃ each independently represent a hydrogen atom, a hydroxyl group or a lower alkyl group or an aryl group that may optionally be substituted with a hydroxyl group; R₄ represents a carboxyl group; R₅ represents a hydrogen atom or a lower alkyl group that may optionally be substituted with a hydroxyl group; R₆ and R₇ each independently represent a hydrogen atom or a lower alkyl group that may optionally be substituted with a hydroxyl group; and, nitrogen atoms in the formula may be in a quaternary form with a lower alkyl group.

Other preferable compounds of the formula (1) and compounds of the formula (1)' are the compounds wherein R₁, R₂ and R₃ each independently represent a hydrogen atom, a hydroxyl group, a lower alkoxy group that may optionally have a substituent, an aryl group that may optionally be substituted with a hydroxyl group, an amino group, a lower alkyl group that may optionally be substituted with a hydroxyl group or an amino group, a guanidino group or a lowr alkyl group that substituted with a guanidino group; R₄ and R₅ each independently represent a hydrogen atom, a hydroxyl group, or a lower alkyl group or an aryl group that may optionally be substituted with a hydroxyl group or an amino group; R₆ and R₇ each independently represent a hydrogen atom, a lower alkyl group that may optionally be substituted with a hydroxyl group, or a lower alkylcarbonyl group; and, nitrogen atoms in the formula may be in a quaternary form with a lower alkyl group.

More preferable compounds of the formula (1) and the formula (1)' are the compounds wherein R₁, R₂ and R₃ each independently represent a hydrogen atom, a hydroxyl group, an aryl group that may optionally be substituted with a hydroxyl group, an amino group, or a lower alkyl group that may optionally be substituted with a hydroxyl group or an amino group; R₄ and R₅ each independently represent a hydrogen atom, a hydroxyl group, or a lower alkyl group that may optionally be substituted with a hydroxyl group; R₆ and R₇ each independently represent a hydrogen atom or a lower alkyl group that may optionally be substituted with a hydroxyl group; and, nitrogen atoms in the formula may be in a quaternary form with a lower alkyl group.

Other further preferable compounds of the formula (1) and compounds of the formula (1)' are the compounds wherein R₁, R₂ and R₃ each independently represent a hydrogen atom, a hydroxyl group, or an aryl group or a lower alkyl group that may optionally be substituted with a hydroxyl group; R₄ and R₅ each independently represent a hydrogen atom, a hydroxyl group, or a lower alkyl group that may optionally be substituted with a hydroxyl group; R₆ and R₇ each independently represent a hydrogen atom or a lower alkyl group that may optionally be substituted with a hydroxyl group; and, nitrogen atoms in the formula may be in a quaternary form with a lower alkyl group.

The most other preferable compounds of the formula (1) and compounds of the formula (1)' are the compounds wherein R₁, R₂ and R₃ each independently represent a hydrogen atom, a hydroxyl group, or an aryl group that may optionally be substituted with a hydroxyl group; R₄ and R₅ each independently represent a hydrogen atom, a hydroxyl group, or an alkyl group having 1 to 3 carbon atoms that may optionally be substituted with a hydroxyl group; R₆ and R₇ each independently represent a hydrogen atom or a lower alkyl group that may optionally be substituted with a hydroxyl group; and, nitrogen atoms in the formula may be in a quaternary form with a lower alkyl group.

The skin conditioner according to the present invention preferably includes a plurality of active ingredients, especially L-arginine (including salts and ions thereof), an ammonium salt (including ions thereof) and/or the compound of the formula (1)' (including salts and ions thereof). In this case, the ratio of the amount of L-arginine to the amount of an ammonium salt and/or the compound of the formula (1)' (the total amount if plurality of the compounds are present) may be varied depending on the kind of disease, age of the patient and severity of disease, and preferably 1000:1 to 1:100, more preferable 100:1 to 1:10 in terms of the weight ratio.

Further, in case the skin conditioner of include an ammonium salt (including ions thereof) and the compound of the formula (1)' (including salts and ions thereof), the ratio of the amount of an ammonium salt to the amount of the compound of the formula (1)' (the total amount if plurality of the compounds are present) may be varied depending on the kind of disease, age of the patient and severity of disease, and preferably 100:1 to 1:100, more preferable 10:1 to 1:10 in terms of the weight ratio. In case all of L-arginine, an ammonium salt and the compound of the formula (1)' are present, weight ratio among the ingredients and preferable weight ratio are determined by combining those of the above two ingredients.

Also, the skin conditioner of the present invention may further include a natural substance preparation. Here, the natural substance preparation may be any substances when the raw materials are natural substances. The natural substance preparation is preferably a plant preparation, more preferably a grain preparation (for example, rice, barley, wheat, adlay, oats, Japanese millet, foxtail millet and Chinese millet), and particularly preferably a rice preparation. Here, a "rice" as a raw material for a rice preparation is not particularly limited. That is, any kinds of rice may be used and any of unpolished rice, polished rice, crushed products thereof, red bran and white bran, for example, may be used as long as they are originating from rice, and germinated rice may also be used. The raw material rice may be treated by crushing, immersing, steaming and roasting, and may also be treated by extracting, disintegrating (with enzymes and malted rice) and fermenting (organic acid fermentation and alcohol fermentation). The most preferred is a rice extract fermented substance.

As an example of the natural substance preparation, the preparation is obtained by hydrating rice, crushed rice or rice bran, reacting by adding amylaze or additionally ptoteasea, adding yeast after the reaction, and subjecting to saccharification and fermentation. In addition, the preparation is obtained by hydrating rice, crushed rice or rice bran, adding at least one of amylaze, ptotease and lipase, heating, and extracting by heating or further repeating these reactions two or more times. Further, the preparation is obtained by fermenting an animal, plant or microbial substance, or adding a fermenting sugar, followed by fermentation. In addition, the preparation is obtained by adding water, as necessary, to the animal, plant or microbial material, adding at least one of amylase, protease and lipase, heating, and extracting by heating or further repeating these reactions two or more times.

Here, while the natural substance preparation may include all or a part of the compounds of the formula (1), in case of an essential ingredient is included in the natural substance preparation, the essential ingredient included in the natural substance preparation may not be added again.

Also, the preparation may further include a moisture retention agent. As an example of the moisture retention agent, the agent contains one or more kinds of substances selected from the group consisting of polyvalent alcohols represented by glycerin, dipropyleneglycol and 1,3-butyleneglycol; sugars represented by sorbitol, maltitol, dextrin, hyaluronic acid and chitosan; mucopolysaccharides and sugar derivatives; polypeptides represented by elastin and collagen; organic acids and their salts represented by pyrrolidone carboxylic acid, citric acid and lactic acid; biopharmaceutical and natural moisture retention agents represented by refined rice wine, rice bran, aloe, glycyrrhizac radix and chamomile; bio-component moisture retention agents represented by vitamins, placental extract, urea, lecithins, phospholipids, ceramides, cholesterols and sphingolipids; and, vegetable extracts, fruit extracts, kelp extracts, enzymes and inorganic salts; and, is one or more substances selected from the group consisting of animal oils, vegetable oils, hydrocarbons, higher alcohols and esters.

Other components which may be added includes drugs, such as one or more kinds of substances selected from the group consisting of bactericidal drugs, wound protective agents, wound healing agents, drugs for suppurative diseases, analgesic, anti-itching, astringent and antiphlogistic agents, immunosuppresants, drugs for parasitic skin diseases, skin softeners, hair agents, vitamin agents and biopharmaceuticals; and the bases, such as one or more kinds of substances selected from the group consisting of astringents, refrigerants, antioxidants, ultraviolet absorbers, ultraviolet dispersants, preservatives, antibiotics, chelating agents, surfactants, foaming agents, stabilizers, penetrants, assistants, pH adjusters, buffers, emulsifiers, opacefiers, fragrances and pigments.

The skin conditioner according to the present invention preferably has an alcoholic level of 0 to 10%(v/v). This alcoholic level is determined in accordance with the analytic method designated by Bureau of Internal Revenue.

The skin conditioner according to the present invention can be used for drugs, quasi-drugs and cosmetics. Particularly preferred is an externally applied preparation. In addition, either dry or wet forms can be adopted. In case of wet forms, active ingredients may be dissolved or dispersed depending on the application.

In case of the use as an externally applied preparation, the skin conditioner is, for example, directly applied to or rubbed on affected part of the skin, or applied on gauze and cloth then applied to or sprayed on the skin. In addition, in case of the use as a washing agent (for example, solid, liquid or foam soap), after lathering up the agent using water or lukewarm water and cleaning the skin, the agent is washed off by water or lukewarm water. The number and amount of applications may be adjusted depending on the kind and severity of disease of the patient. In case of the use as an externally applied preparation, it is preferably applied once to several times per day and the amount per application should be such that an active ingredient is preferably 0.001 to 1000 µg/cm² or more, more preferably 0.01 to 1000 µg/cm² or more.

Use of the skin conditioner conditions the epidermis, and the epidermis and the dermis. This use can give not only moisture retention effects, but also enhance moisture retention functions of the skin, and also restore fineness and moistness of the skin. Particularly, it is effective against dry skin symptoms, such as symptoms selected from atopic skin, dry or rough skin, aged skin, ichthyosis, dry skin, chapped skin, asteatosis, xeroderma, dry eczema, facial dry eczema and progressive volar keratoderma, and/or erythema, sclerosis and cornification, cracking, scaling, wrinkles, itching and scratched scars; skin aging symptoms, such as wrinkles and decreased skin tightness and elasticity; skin damage caused by ultraviolet rays, such as spots and freckles; skin disorders arising from the epidermis, such as turnover abnormalities, fineness and moistness; physicochemical skin disorders, such as wound healing, cuts, burns and floor burns; biological skin disorders, such as athlete's foot and skin infections; and dermatitis and eczema, such as inflammatory cornification disorders (psoriasis). Especially, it is effective in the prevention and treatment of skin diseases such as atopic dermatitis, dry skin symptoms, pruritis, frostbite, cracking, chapped skin, skin aging symptoms, skin damage caused by ultraviolet rays, darkening, blackening, skin disorders arising in the epidermis, physicochemical skin disorders, skin symptoms caused by the use of water, soap, detergents, surfactants or solvents, adverse side effects of externally applied skin preparations, biological skin disorders, dermatitis, eczema and other skin diseases.

Especially, in case the skin conditioner is used as an agent for restoring the skin's barrier mechanism and function, its application instantly acts on the skin and its effect sustains after the application is discontinued. That is, it can demonstrate its effectiveness in, for example, sustaining moisture retention duration over 2 hours by one application, enhancing moisture retention ability instantly by one application, not decreasing enhanced moisture retention ability when applied for a long time after discontinuation of application, improving artificially induced chapped skin to a healthier skin compared to its original state before a chapped skin is induced, and inhibiting allergic reaction by preventing infiltration of house dusts.

### Examples

### Test Example 1 (Test for conditioning the dermal)

A collagen production recovery test was conducted on damaged fibroblasts.

Fibroblasts are cells that compose the dermis which is on the inside of the skin epidermis. Collagen produced by fibroblasts accounts for approximately 70% of the weight of the dermis, and gives the skin tightness, elasticity and flexibility. In addition, when the skin becomes injured and so forth, it also fulfills the role of the regenerative function of the skin. As the skin ages, the amount of collagen decreases dramatically. Consequently, the skin loses its tightness and elasticity, and wounds are known to heal more slowly. In addition, even in the absence of aging of the skin, the ability to produce collagen decreases due to various causes such as routine exposure to ultraviolet rays and radiation, and the generation of active oxygen.

Samples:

### Example 1

1% aqueous solution of L-arginine (Nakarai Tesuku)

### Example 2

1% aqueous solution of ethanolamine (Nakarai Tesuku)

### Example 3

After crushing 1 kg of rice with a crusher, 250 g of water were mixed in well while spraying followed by allowing to stand for 30 minutes. Next, the rice was boiled for 60 minutes followed by the addition of 2000 mL of water. Moreover, after adding 7.5 g each of α-amylase and β-amylase, the mixture was allowed to stand for 10 hours at 55°C. Next, after gradually raising the temperature and boiling for 5 minutes, the mixture was cooled to 50°C followed by the addition of 30 g of citric acid, 8 g of acidic protease and 8 g of acidic carboxypeptidase and allowing to react for 24 hours. After completion of the reaction, the mixture was cooled to 20°C followed by the addition of 200 g of malted rice (Aspergillus oryzae) and pre-cultured Saccharomyces cereviciae culture broth, and fermenting at 20-25°C for 20 days.

Following completion of fermentation, the mixture was press-filtered to obtain 2700 mL of filtrate. Next, 500 mL of activated charcoal were packed into a column and the filtrate was passed through the column. The resulting effluent was collected, concentrated to two-fold with evaporator and water was added thereto to obtain 2700 mL of product containing 1934 mg/L of L-arginine and 162 mg/L of ethanolamine. (The concentration of L-arginine was approximately 0.2%, and that of ethanolamine was approximately 0.02%.)

### Mixture of samples of Example 1 and Example 2:

### Mixture of samples of Example 1 and 2 with the product of Example 3:

### Test Method:

Six to eight subcultures of normal human skin fibroblasts (Physicochemical Research Institute, Cell Development Bank NBIRGB) were used in the test.

Hypoxanthine at a final concentration of 50 µM and 34.5 mU/dish of xanthine oxidase were added to the culture broth to generate active oxygen and lower the collagen production ability of the cells.

Measurement of collagen production ability of a confluent in the steady state was performed according to the method of Webster et al. based on the uptake of ³H-proline into the produced collagen. Furthermore, the samples were mixed with the cells to a final concentration of 3.3% (taking 1% to be 100% for a 1% aqueous solution) and after incubating at 37°C for 24 hours and 5% CO₂, the ³H activity taken up into the collagen in the cells was measured.

### Reference: Principle of Measurement of Collagen Production Ability

Since proline is a main component of the amino acids that compose collagen, fibroblasts are cultured in a medium containing ³H-proline, and the ³H activity taken up into collagen in the cells is measured. Units are in d.p.m., and represent the number of daltons of radioactivity released per minute.

### Test Results:

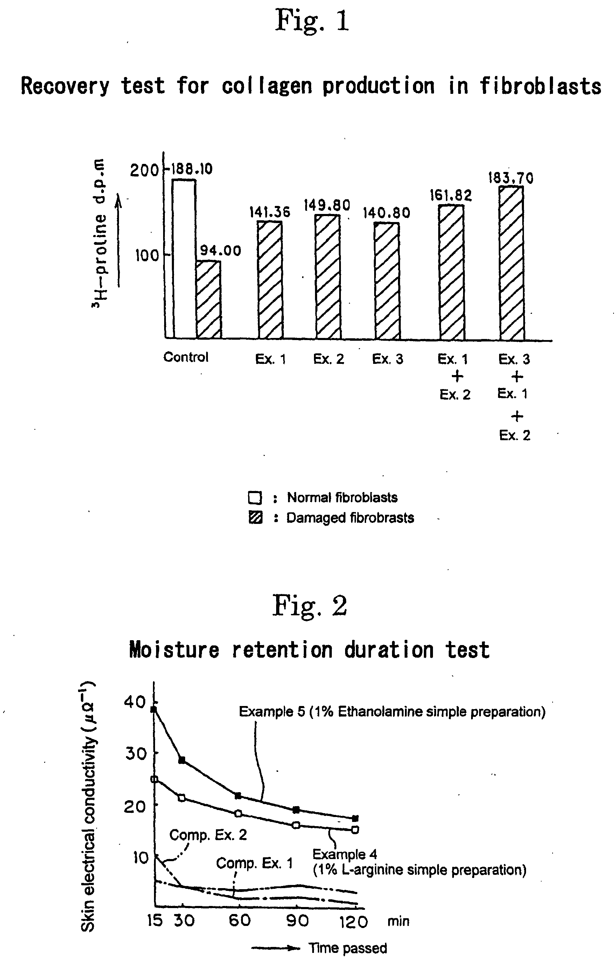

As shown in Fig. 1, according to the results of a collagen production recovery test, the collagen production ability of fibroblasts damaged by active oxygen was determined to be significantly improved by L-arginine and ethanolamine. Although L-arginine and ethanolamine are contained in the product of Example 3, since the amounts are excessively small, production was nearly equal to Example 1. When 1% L-arginine and 1% ethanolamine were further added to the product of Example 3, production nearly completely recovered.

Namely, although remarkable recovery is observed with L-arginine or ethanolamine alone, if both L-arginine and ethanolamine are present and their amounts are increased, the collagen production ability of damaged fibroblasts can be nearly completely restored to its original normal level.

### Test Example 2 (Test for conditioning the skin's barrier mechanism and function, particularly for conditioning the corneal layer of the epidermis)

A moisture retention duration test was conducted.

Moisture retention refers to the peak of the amount of skin moisture (skin electrical conductivity) 15 minutes after application, while moisture retention duration refers to the integral value of a curve indicated by the amount of skin moisture (skin electrical conductivity) from 30 minutes to 120 minutes after application.

### Samples:

### Example 4 (1% L-arginine simple preparation)

| | |
|---|---|
| 1% aqueous solution of L-arginine | |
| (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water [pH adjustor (ex. hydrochloric acid and caustic soda) was suitably added in suitable amount to all the ingredients used in the following Examples 2 to 19 besides this Example so as to adjust pH 6 to 6.8].

### Example 5 (1% Ethanolamine simple preparation)

| | |
|---|---|
| 1% aqueous solution of Ethanolamine | |
| (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 6 (0.2% L-arginine + 0.02% Ethanolamine + simple preparation)

| | |
|---|---|
| Example 3 | 90 mL |
| 95% Ethanol | 2.00 mL |
| Parabene | 0.18 g |
| Purified soy bean lecithin | 0.05 g |

Make up a final amount of 100.00 g by addition of purified water.

### Comparative Example 1 (simple preparation)

| | |
|---|---|
| 95% Ethanol | 2.00 mL |
| Parabene | 0.18 g |
| Purified soy bean lecithin | 0.05 g |

Make up a final amount of 100.00 g by addition of purified water.

### Comparative Example 2 (Hyaluronic acid + simple preparation)

| | |
|---|---|
| Sodium hyaluronate (2) | 1.00 g |
| 95% Ethanol | 2.00 mL |
| Parabene | 0.18 g |
| Purified soy bean lecithin | 0.05 g |

Make up a final amount of 100.00 g by addition of purified water.

### Panelists: 5 healthy volunteers

Test Method: Each sample was applied to the side of the forearm of the panelists (4 x 4 cm²) followed by measurement of epidermal keratin moisture content at 15, 30, 60, 90 and 120 minutes after application.

Keratin contains salts, amino acids and other electrolytes in addition to moisture. Consequently, although current does not flow through pure water, since electrolytes are contained in keratin in the skin, current flows corresponding to the amount of moisture present if moisture is present. The parameter that is actually measured is electrical conductivity, which is the inverse of the resistance that composes impedance.

### Measurement Method:

(1) The test site is washed with soap.
(2) The test site is exposed in a constant temperature and constant humidity room at a temperature of 20°C and humidity of 50%, and the skin is allowed to reach a steady state by allowing the panelists to rest quietly starting 60 minutes before measurement.
(3) The moisture content of keratin at the test site is measured and used as the value before application.
(4) After uniformly applying 0.03 mL aliquots of sample to the test site four times, the sample is gently wiped off with gauze.
(5) The moisture content of the keratin at the test site 15, 30, 60, 90 and 120 minutes after application, and that of keratin at a site at which sample is not applied as a control, were measured.

Numerical values obtained by subtracting the value before application and value of the site where sample was not applied from the keratin moisture content for each measurement time were taken to represent skin moisture content.

### Test Apparatus:

### SKICON-200 [IBS Epidermal Keratin Moisture Measuring System (3.5 MHz high-frequency conductivity measuring system)]

### Test Results:

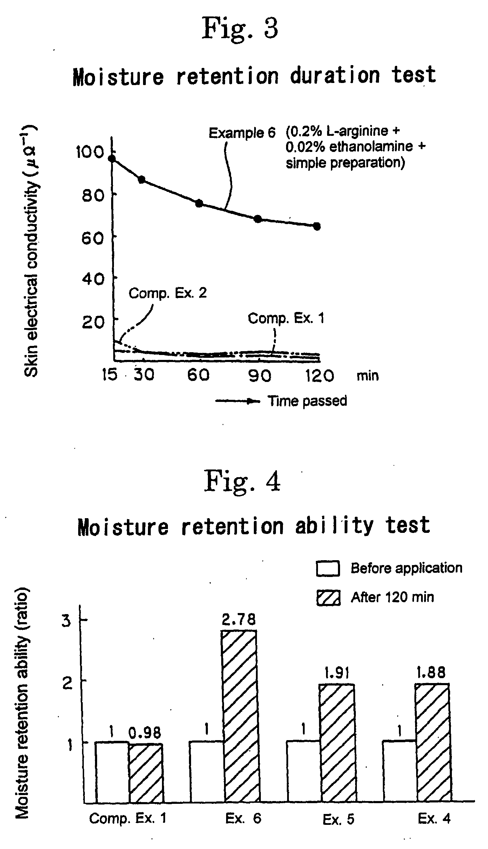

The results of the moisture retention duration test are as shown in Figs. 2 and 3.

Although peak values rose even at 15 minutes after application and moisture retention effects were remarkable for Examples 4, 5 and 6, moisture retention continued beyond 30 minutes and lasted for 2 hours. Although continuation of moisture retention was observed with either L-arginine or ethanolamine alone, when both substances were present, moisture retention duration was enhanced more than when either substance was used alone even at lower concentrations.

On the other hand, although peaks were observed after 15 minutes in the case of Comparative Examples 1 and 2, moisture content returned to its original level after 30 minutes, and continuation of moisture retention was not observed at all.

### Test Example 3 (Test for conditioning the skin's harrier mechanism and function, particularly for conditioning the corneal layer of the epidermis)

A moisture retention ability test was conducted as an indicator of the state of skin health.

### Samples:

### Example 4 (L-arginine + simple preparation)

### Example 5 (Ethanolamine + simple preparation)

### Example 6 (L-arginine + ethanolamine + simple preparation)

### Example 7 (L-arginine + ethanolamine + body soap preparation)

| | |
|---|---|
| Example 3 | 20 mL |
| Lauric acid | 2.50 g |
| Myristic acid | 7.50 g |
| Palmitic acid | 2.50 g |
| Oleic acid | 2.50 g |
| Lauroyldiethanolamide | 5.00 g |
| Glycerin | 20.00 g |
| Parabene | 0.20 g |
| Caustic potash | 3.60 g |
| Edetate | 0.20 g |
| Fragrance | q.s. |

Make up a final amount of 100.00 g by addition of purified water.

### Comparative Example 1 (simple preparation)

### Comparative Example 3

| | |
|---|---|
| Lauric acid | 2.50 g |
| Myristic acid | 7.50 g |
| Palmitic acid | 2.50 g |
| Oleic acid | 2.50 g |
| Lauroyldiethanolamide | 5.00 g |
| Glycerin | 20.00 g |
| Parabene | 0.20 g |
| Caustic potash | 3.60 g |
| Edetate | 0.20 g |
| Fragrance | q.s. |

Make up a final amount of 100.00 g by'addition of purified water.

### Panelists: 4 healthy volunteers

### Measurement Method:

(1) The test site is washed with soap.
(2) The test site is exposed in a constant temperature and constant humidity room at a temperature of 20°C and humidity of 50%, and the skin is allowed to reach a steady state by allowing the panelists to rest quietly starting 60 minutes before measurement.
(3) The moisture content of keratin at the test site is measured.
(4) 0.03 mL of distilled water is placed over the test site and wiped off with gauze 10 seconds later followed by measurement of keratin moisture content at the test site immediately, 30, 60, 90 and 120 seconds after wiping off.
(5) 0.03 mL aliquots of sample are applied to the test site three times and allowed to stand for 15 minutes.
(6) The test site is washed well.
(7) After 120 minutes, keratin moisture content is measured after 120 seconds by performing the same procedure as in step (4).

Moisture retention ability is determined in the manner indicated below.

Moisture retention ability (%) = [Keratin moisture content 30-120 seconds after moisture loading/Keratin moisture content immediately after moisture loading] x 100

It should be noted that, moisture retention ability (ratio) was expressed as the ratio obtained when the moisture retention ability before washing (%) is given a value of 1.

Test Apparatus: Same as Test Example 2.

### Test Results:

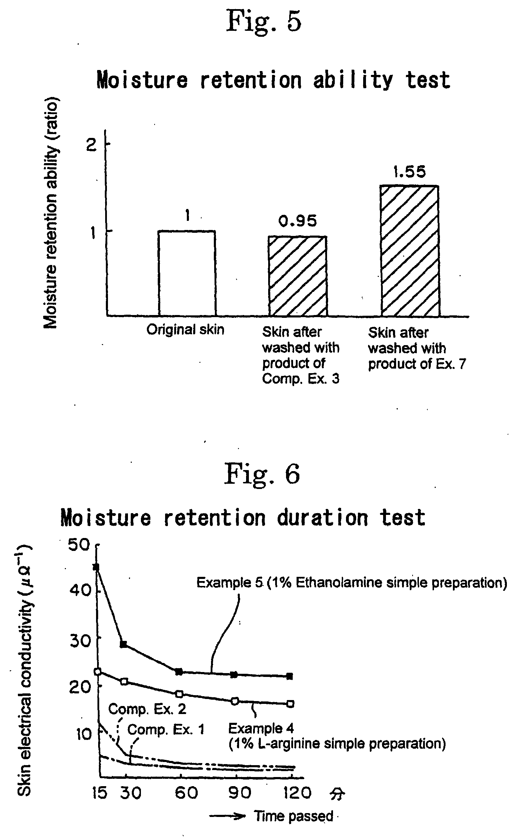

The results of the moisture retention ability test are as shown in Figs. 4 and 5.

Although there was no increase whatsoever in moisture retention ability, which represents the health of the skin, observed for Comparative Example 1, the moisture retention ability 2 hours after application in Examples 4, 5 and 6 increased considerably as compared with the moisture retention ability before application. When the moisture retention ability before application is taken to have a value of 1, although that of Examples 4 and 5 is nearly two times greater, in Example 6, the moisture retention ability increased to nearly three times greater.

In the case of washing with the product of Comparative Example 3, although moisture retention ability decreases as compared with that before washing, washing with Example 7 resulted in an increase in moisture retention ability as compared with before washing.

### Test Example 4 (Test for conditioning the skin's barrier mechanism and function, particularly for conditioning the corneal layer of the epidermis)

A moisture retention duration test was conducted on persons with chapped skin.

### Samples:

### Example 4 (L-arginine + simple preparation)

### Example 5 (Ethanolamine + simple preparation)

### Example 6 (L-arginine + ethanolamine + simple preparation)

### Comparative Example 1

### Comparative Example 2

### Panelists: 6 volunteers with chapped skin

### Test Method:

Each sample was applied to the side of the forearm of the panelists (4 x 4 cm²) followed by measurement of epidermal keratin moisture content at 15, 30, 60 and 120 minutes after application.

Measurement Method: Same as measurement method of Test Example 2.

Determination of Skin Moisture Content: Same as Test Example 2.

Test Apparatus: Same as Test Example 2.

### Test Results:

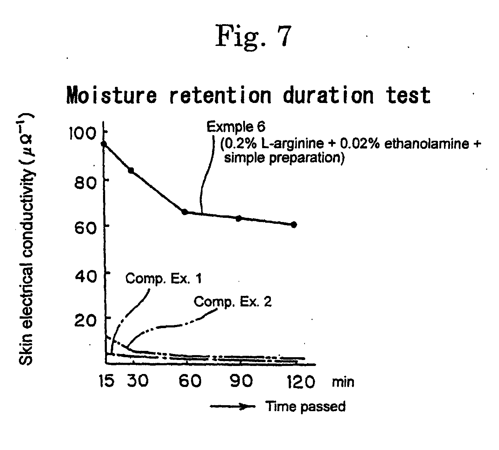

As shown in Figs. 6 and 7, although the peaks increased and moisture retention effects were remarkable after 15 minutes for Examples 4, 5 and 6, moisture retention continued beyond 30 minutes and lasted for 2 hours. Although this continuation of moisture retention was also observed in Examples 4 and 5, the duration of moisture retention was even greater in Example 6 that contained both L-arginine and ethanolamine.

In Comparative Example 2, although a peak was observed after 15 minutes and moisture retention effects were observed, moisture content returned to its original level after 30 minutes, and continuation of moisture retention with respect to chapped skin was not observed at all.

### Test Example 5 (Test for conditioning the skin's barrier mechanism and function, particularly tests for conditioning the corneal layer of the epidermis, for conditioning the epidermal keratocytes, on effects for promoting the production of the healthy corneal layer of the epidermis, and for normalizing cell differentiation)

Chapped skin was induced artificially and a recovery test for moisture retention ability was conducted to observe the effects against damaged skin (skin susceptible to both external and internal irritation).

### Samples:

### Example 6 (L-arginine + ethanolamine + simple preparation)

### Example 8 (L-arginine + ethanolamine + cream preparation)

| | |
|---|---|
| Example 3 | 40 mL |
| 1,3-Butyleneglycol | 6.00 g |
| Concentrated glycerin | 6.00 g |
| Methylpolysiloxane | 6.00 g |
| Stearic acid | 3.00 g |
| Cetanol | 3.00 g |
| Cetyl 2-ethylhexanoate | 6.00 g |
| Squalene | 6.00 g |
| Sucrose fatty acid ester | 3.00 g |
| Natural vitamin E | 0.30 g |
| Sodium casein | 1.50 g |
| Disodium edetate | 0.03 g |
| Parabene | 0.30 g |

Make up a final amount of 100.00 g by addition of purified water.

### Comparative Example 1

### Comparative Example 2

### Comparative Example 4 (Cream preparation)

| | |
|---|---|
| 1,3-Butyleneglycol | 6.00 g |
| Concentrated glycerin | 6.00 g |
| Methylpolysiloxane | 6.00 g |
| Stearic acid | 3.00 g |
| Cetanol | 3.00 g |
| Cetyl 2-ethylhexanoate | 6.00 g |
| Squalene | 6.00 g |
| Sucrose fatty acid ester | 3.00 g |
| Natural vitamin E | 0.30 g |
| Sodium casein | 1.50 g |
| Disodium edetate | 0.03 g |
| Parabene | 0.30 g |

Make up a final amount of 100.00 g by addition of purified water.

### Panelists: 4 healthy volunteers

Test Method: After inducing chapped skin by treating a healthy site of the skin for 30 minutes with 5% SDS, each sample was applied twice per day, and the instantaneous moisture retention ability before application and from 1 day to 2 weeks after application was measured according to the same method as Test Example 3.

Chapped Skin Inducing Method: A glass cylinder was placed on the test site and fixed in position with tape. Next, 10 mL of 5% SDS (sodium lauryl sulfate) was poured into the glass cylinder to perform chapped skin treatment for 30 minutes while stirring occasionally.

Finally, the SDS was suctioned out of the glass cylinder and the glass cylinder was removed.

Test Apparatus: Same as Test Example 2.

### Test Results:

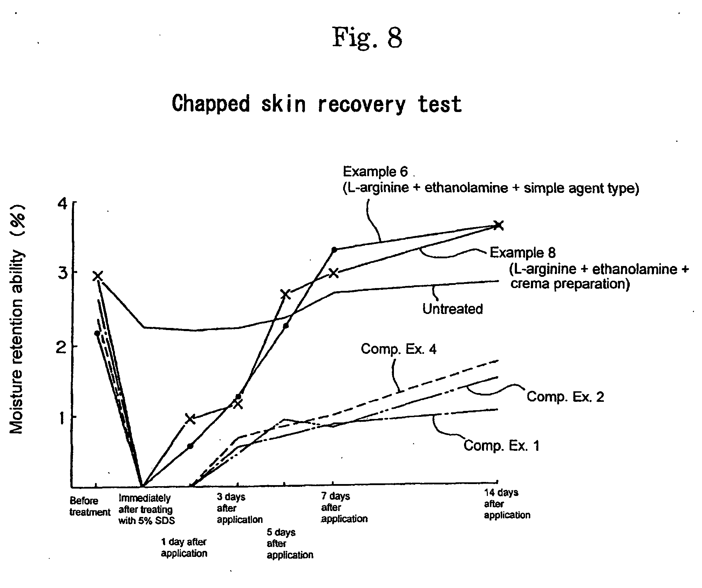

According to the results of the chapped skin recovery test (Fig. 8), only natural recovery of the skin was observed with the simple preparation form, the typical moisture retention agent, hyaluronic acid, and a typical cream preparation not containing L-arginine or ethanolamine, and chapped skin improvement effects were not observed. On the other hand, in the case of Examples 6 and 8, moisture retention ability increased significantly in comparison with the control group at 3, 5 and 7 days after the start of application, and moisture retention ability was higher than the untreated site (healthy site) starting at 5 days after the start of application.

In this manner, Examples 6 and 8 were clearly demonstrated to rapidly restore damaged skin and improve the skin to healthy skin to a greater extent than the untreated site.

The present invention was proven to rapidly restore damaged skin in a short period of time, enable the skin to reach a state that is healthier than its original state, and have effects that improve the skin to its healthiest state. On the basis of these findings, the present invention was proven to act on chapped skin itself and condition it, be able to prevent skin diseases caused by chapped skin, and demonstrate chapped skin therapeutic effects.

### Test Example 6 (Test for conditioning the skin's barrier mechanism and function)

A clinical test was conducted on dry eczema, xeroderma and facial dry eczema patients to observe the therapeutic effects on skin diseases produced by skin conditioning, and those effects were evaluated in terms of the severity score of itchiness, sclerosis, cornification, scaling, cracking, erythema, dryness and wrinkles, as well as overall improvement (usefulness) with respect to each disease.

### Samples:

### Example 9 (L-arginine + milky liquid preparation)

| | |
|---|---|
| 1% aqueous solution of L-arginine | |
| (Nakarai Tesuku) | 0.10 g |
| 1,3-Butyleneglycol | 10.00 g |
| Concentrated glycerin | 1.00 g |
| Stearic acid | 0.50 g |
| Myristic acid | 0.50 g |
| Bleached beeswax | 0.50 g |
| Tri-2-ethylhexanoate glycerin | 4.80 g |
| Octyldodecylmyristic acid | 2.00 g |
| Squalene | 1.00 g |
| Sucrose fatty acid ester | 0.60 g |
| Xanthane rubber | 0.10 g |
| Natural vitamin E | 0.10 g |
| Sodium casein | 0.30 g |
| Citric acid | q.s. |
| Disodium edetate | 0.02 g |
| Parabene | 0.20 g |

Make up a final amount of 100.00 g by addition of purified water.
- Panelists:: 3 patients with dry eczema
2 patients with xeroderma
2 patients with facial dry eczema

### Test Sites:

Sites having symptoms suitable for evaluation and sites that can be compared to the left or right or above or below (comparison with non-application).

External Application Method: Simple application twice per day (morning and evening).

### Application Period: 3 weeks

### Evaluation Items:

### Evaluation items consisted of:

(1) Itchiness
(2) Scierosis/cornification
(3) Scaling
(4) Cracking
(5) Erythema
(6) Dryness
(7) Wrinkles

### Evaluation Method:

The evaluation items were evaluated according to the following four levels of a severity score as determined by visual examination.
3: Advanced symptoms
2: Moderate symptoms
1: Mild symptoms
0: No symptoms or symptoms disappeared

In addition, overall improvement (usefulness) was evaluated according to the following four levels:
Extremely useful
Useful
Somewhat useful
Not useful

### Test Results:

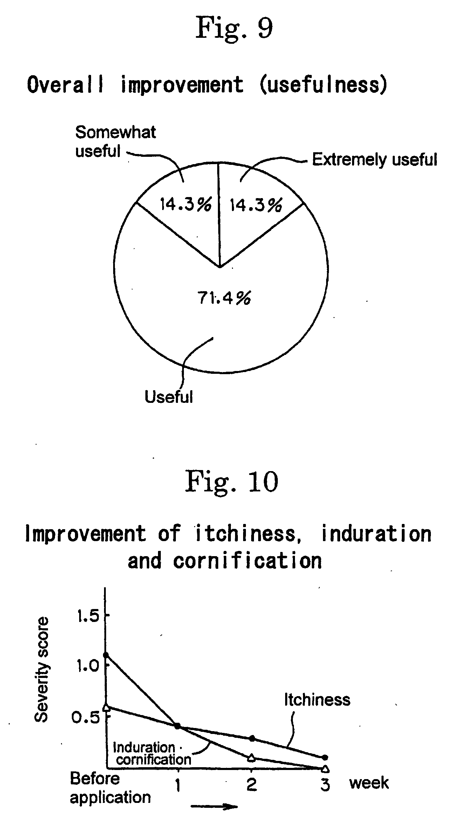

The results for overall improvement (usefulness) are as shown in Fig. 9. When Example 9 product was used in patients with dry eczema, xeroderma and facial dry eczema, the results demonstrated overall improvement of 100%, a high degree of usefulness was obtained, and Example 9 was recognized to be extremely useful against these diseases.

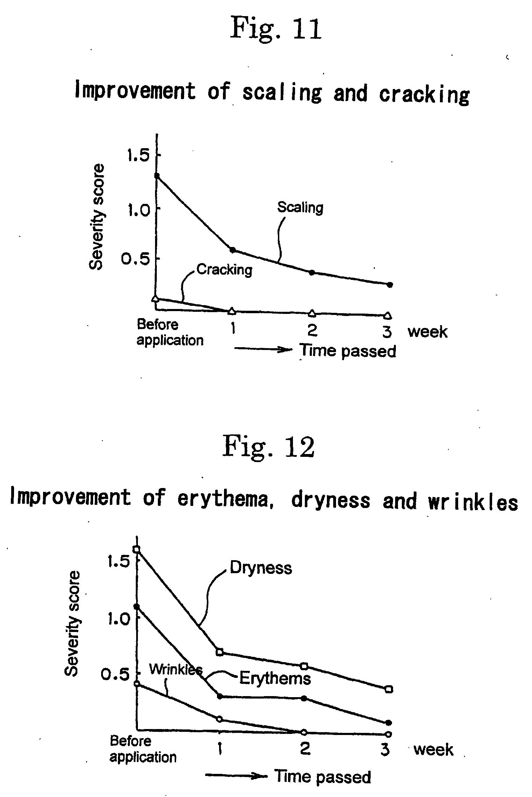

Fig. 10 shows the changes in severity scores for itchiness, sclerosis and cornification. Fig. 11 shows the changes in severity scores for scaling and cracking. Fig. 12 shows the changes in severity scores for erythema, dryness and wrinkles. All effects appeared rapidly, and all symptoms were alleviated considerably after 1 week of use. Favorable improvement effects were also observed after 1 week, and nearly all symptoms had either been alleviated or disappeared after 3 weeks. It should be noted that, there were no adverse side effects observed at all, there were no cases of relapse after use was discontinued, and the patients were completely healed.

In this manner, the present invention is able to improve symptoms observed in skin diseases such as itchiness, sclerosis, cornification, scaling, cracking, erythema, dryness and wrinkles through conditioning of the skin.

### Test Example 7 (Test for conditioning the skin's barrier mechanism and function)

A clinical test was conducted on asteatosis, xeroderma, facial dry eczema and progressive volar keratoderma patients to observe the therapeutic effects on skin diseases produced by skin conditioning, and those effects were evaluated in terms of the severity score of itchiness, sclerosis, cornification, scaling, cracking, erythema, dryness and wrinkles, as well as overall improvement (usefulness) with respect to each disease.

### Samples:

### Example 10 (L-arginine + ethanolamine + milky liquid preparation)

| | |
|---|---|
| Example 3 | 35 mL |
| 1,3-Butyleneglycol | 10.00 g |
| Concentrated glycerin | 1.00 g |
| Stearic acid | 0.50 g |
| Myristic acid | 0.50 g |
| Bleached beeswax | 0.50 g |
| Tri-2-ethylhexanoate glycerin | 4.80 g |
| Octyldodecylmyristic acid | 2.00 g |
| Squalene | 1.00 g |
| Sucrose fatty acid ester | 0.60 g |
| Xanthane rubber | 0.10 g |
| Natural vitamin E | 0.10 g |
| Sodium casein | 0.30 g |
| Citric acid | q.s. |
| Disodium edetate | 0.02 g |
| Parabene | 0.20 g |

Make up a final amount of 100.00 g by addition of purified water.

| | |
|---|---|
| Panelists: Asteatosis patients | 6 |
| Xeroderma patients | 4 |
| Facial dry eczema patients | 4 |
| Progressive volar keratoderma patients | 5 |

### Test Sites:

Sites having symptoms suitable for evaluation and sites that can be compared to the left or right or above or below (comparison with non-application).

External Application Method: Simple application once per day (morning and evening).

### Application Period: 3 weeks

### Evaluation Items:

Evaluation items consisted of:
(1) Itchiness
(2) Sclerosis/cornification
(3) Scaling
(4) Cracking
(5) Erythema
(6) Dryness
(7) Wrinkles

### Evaluation Method:

The evaluation items were evaluated according to the following four levels of a severity score as determined by visual examination.
3: Advanced symptoms
2: Moderate symptoms
1: Mild symptoms
0: No symptoms or symptoms disappeared

In addition, overall improvement (usefulness) was evaluated according to the following four levels:
Extremely useful
Useful
Somewhat useful
Not useful

### Test Results:

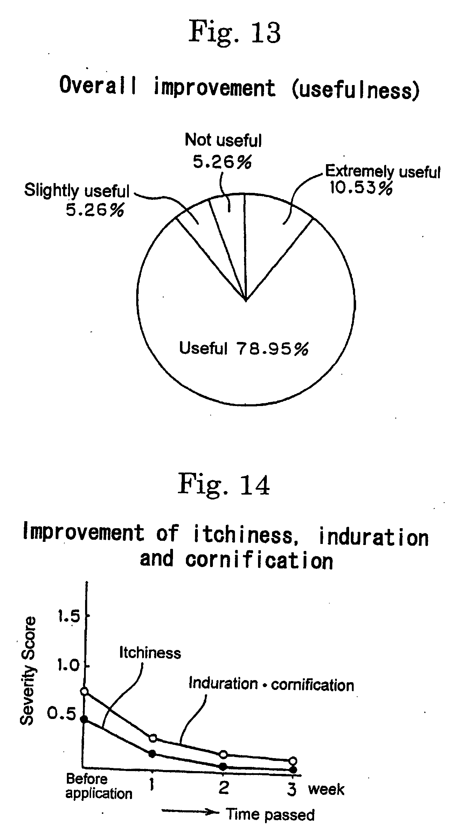

The results for overall improvement (usefulness) are as shown in Fig. 13.

When Example 10 was used in asteatosis, xeroderma, facial dry eczema and progressive volar keratoderma patients, it demonstrated overall improvement of 94.74%, a high degree of usefulness was obtained, and Example 10 was observed to be extremely useful against these diseases.

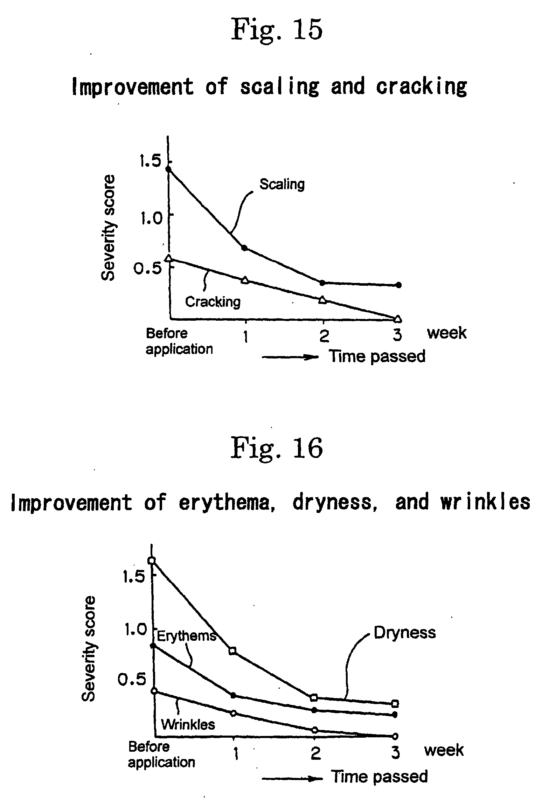

Fig. 14 shows the changes in severity scores for itchiness, sclerosis and cornification. Fig. 15 shows the changes in severity scores for scaling and cracking. Fig. 16 shows the changes in severity scores for erythema, dryness and wrinkles. All effects appeared rapidly, and all symptoms were alleviated considerably after 1 week of use. Favorable improvement effects were also observed after 1 week, and nearly all symptoms had either been alleviated or disappeared after 3 weeks. It should be noted that, there were no adverse side effects observed at all, there were no cases of relapse after use was discontinued, and the patients were completely healed.

In this manner, the present invention is able to improve symptoms observed in skin diseases such as itchiness, sclerosis, cornification, scaling, cracking, erythema, dryness and wrinkles through conditioning of the skin.

### Test Example 8 (Test for conditioning the dermal)

Guinea pigs were irradiated with ultraviolet light, a phlogogenic factor, followed by histological examination of the degree of inflammatory changes in epidermal tissue and dermal tissue to observe the preventive and therapeutic effects on inflammation and photoinflammation.

### Samples:

### Example 6 (L-arginine + ethanolamine + simple preparation)

### Comparative Example 1 (simple preparation)

### Experimental Animals: Guinea pigs, 5

### Test Sites:

### Shaved back of guinea pigs (comparison with simple preparation)

### Test Method:

The backs of the experimental animals were shaved and hair was removed with a depilatory cream three days before irradiation with ultraviolet light.

The test site was irradiated with ultraviolet light, and application of samples was started immediately after irradiation.

In order to make a histological evaluation of inflammation caused by irradiation with ultraviolet light, biopsies were performed with a 6 mm disposable punch on days 7 and 14 after irradiation, the specimens were immersed in 10% neutral formalin solution and fixed followed by preparing tissue sections.

Application Method: Simple application twice per day after irradiation (morning and evening).

### Application Period: 2 weeks

### Evaluation Method:

Using keratin hyperplasia and parakeratosis as indicators of inflammatory changes of epidermal tissue, and cellular infiltration and vasodilation as indicators of inflammatory changes in dermal tissue, the tissue sections were observed and evaluations were made according to the following five levels of a severity score (inflammation intensity).

### Severity Score

4: Advanced symptoms
3: Moderate symptoms
2: Mild symptoms
1: Slight symptoms
0: No symptoms or symptoms disappeared

### Test Results:

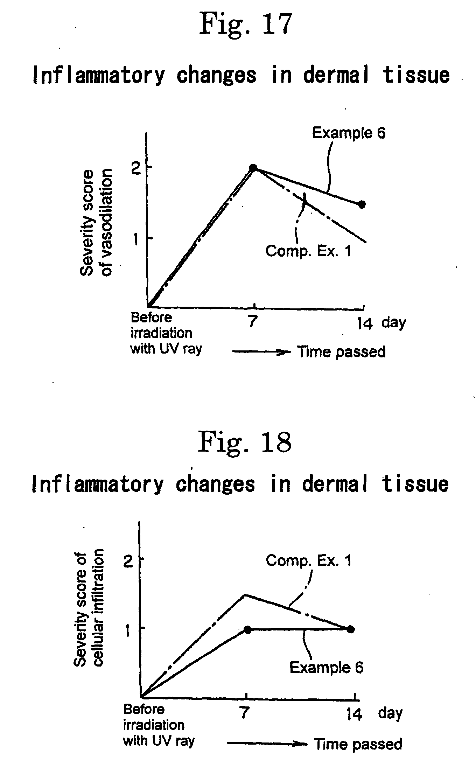

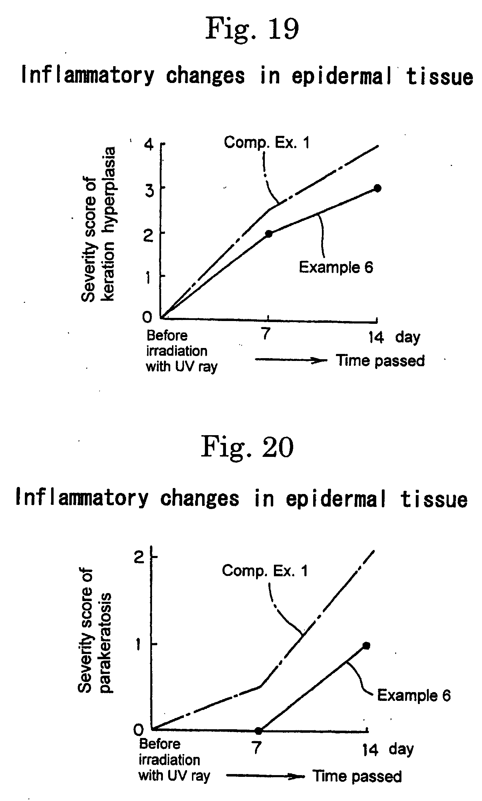

The test results are as shown in Figs. 17, 18, 19 and 20. Example 6 of the present invention was clearly demonstrated to have an effect that heals vasodilation in the early stage of the occurrence of inflammation in dermal tissue, and was also observed to not only have a therapeutic effect in the early stage, but also a preventive effect that prevents full-scale onset of inflammation. In addition, it was also clearly shown to rapidly heal cellular infiltration, which is a symptom of inflammation in the dermis. Furthermore, keratin hyperplasia and parakeratosis, which are abnormalities in the epidermis accompanying inflammation, were also observed to be alleviated.

On the basis of these findings, inflammation and photoinflammation were clearly demonstrated to be prevented and healed by skin conditioning.

### Test Example 9 (Test for conditioning the skin's harrier mechanism and function, particularly tests for conditioning the corneal layer of epidermis, and preventing, preventing exacerbation of and treating atopic dermatitis)

A 2-hour moisture retention duration test was performed on atopic skin.
Panelists: 7 persons with atopic skin
Test Method: Same as Test Example 2
Measurement Method: Same as Text Example 2
Test Apparatus: Same as Test Example 2
The samples were as shown below.

### Example 4 (1% L-arginine simple preparation)

| | |
|---|---|
| 1% aqueous solution of L-arginine | |
| (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 5 (1% Ethanolamine simple preparation)

| | |
|---|---|
| 1% aqueous solution of Ethanolamine | |
| (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 6 (0.2% L-arginine + 0.02% Ethanolamine + simple preparation)

| | |
|---|---|
| Example 3 | 90 mL |
| 95% Ethanol | 2.00 mL |
| Parabene | 0.18 g |
| Purified soy bean lecithin | 0.05 g |

Make up a final amount of 100.00 g by addition of purified water.

### Example 11 (1% 2-Methoxyethylamine simple preparation)

| | |
|---|---|
| 2-Methoxyethylamine | |
| (Tokyo Kasei Kogyo) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 12 (1% O-Phosphorylethanolamine simple preparation)

| | |
|---|---|
| O-Phosphorylethanolamine | |
| (Tokyo Kasei Kogyo) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 13 (1% 2-Ethylaminoethanol simple preparation)

| | |
|---|---|
| 2-Ethylaminoethanol | |
| (Tokyo Kasei Kogyo) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 14 (1% Diethanolamine simple preparation)

| | |
|---|---|
| Diethanolamine | |
| (Mitsui Toatsu Chemicals) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 15 (1% 2-Dimethylaminoethanol simple preparation)

| | |
|---|---|
| 2-Dimethylaminoethanol | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 16 (1% Choline simple preparation)

| | |
|---|---|
| Choline (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 17 (1% 2-Amino-hydroxymethyl-1,3-propanediol simple preparation)

| | |
|---|---|
| 2-Amino-hydroxymethyl-1,3-propanediol | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 18 (1% Noradrenaline simple preparation)

| | |
|---|---|
| Noradrenaline (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 19 (1% Phenethylamine simple preparation)

| | |
|---|---|
| Phenethylamine (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 20 (1% Ethylenediamine simple preparation)

| | |
|---|---|
| Ethylenediamine (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 21 (1% Taurine simple preparation)

| | |
|---|---|
| Taurine (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 22 (1% Phosphatidylethanolamine simple preparation)

| | |
|---|---|
| Phosphatidylethanolamine | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 23 (1% N-(2-Hydroxyethyl)acetamide simple preparation)

| | |
|---|---|
| N-(2-Hydroxyethyl)acetamide | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 24 (1% 2-(Metylamino)ethanol simple preparation)

| | |
|---|---|
| 2-(Metylamino)ethanol (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 25 (1% 2-Anilinoethanol simple preparation)

| | |
|---|---|
| 2-Anilinoethanol | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 26 (1% 2-(Benzylamino)ethanol simple preparation)

| | |
|---|---|
| 2-(Benzylamino)ethanol | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 27 (1% 3-Amino-1-propanol simple preparation)

| | |
|---|---|
| 3-Amino-1-propanol | |
| (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 28 (1% 2-Amino-1-butanol simple preparation)

| | |
|---|---|
| 2-Amino-1-butanol (Kanto Kagaku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 29 (1% Putrescine simple preparation)

| | |
|---|---|
| Putrescine (Sigma Chemical) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 30 (1% DL-Pyroglutamic acid simple preparation)

| | |
|---|---|
| DL-Pyroglutamic acid | |
| (Tokyo Kasei Kogyo) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 31 (1% Triethanolamine simple preparation)

| | |
|---|---|
| Triethanolamine | |
| (Mitsui Toatsu Chemicals) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 32 (Rice preparation containing 0.03% L-arginine)

1 kg of unpolished rice was crushed with a crusher. After adding 3000 mL of water, 7.5 g of α-amylase, 8 g of protease and 8 g of peptidase and heating to 55°C, the mixture was allowed to stand for 10 hours while holding at that temperature. Next, the temperature was gradually raised and extraction was performed by boiling for 5 minutes. After cooling to 20°C, the mixture was press-filtered and the pH of the filtrate was lowered to 3.3 by addition of citric acid. 8 g of acidic protease and 8 g of acidic carboxypeptidase were added followed by allowing to react for 10 hours at 55°C.

Next, the mixture was heated to 70°C and then filtered after cooling to obtain 2700 mL of product containing 354 mg/L of L-arginine.

### Example 33 (Rice preparation containing 0.03% L-arginine + simple preparation)

| | |
|---|---|
| Example 32 (containing 0.03% L-arginine from rice) | 90.00 mL |
| 95% Ethanol | 2.00 mL |
| Parabene | 0.18 g |
| Purified soy bean lecithin | 0.05 g |

Make up a final amount of 100.00 g by addition of purified water.

### Example 34 (1% 2-Methoxyethylamine + rice preparation containing 0.03% L-arqinine + simple preparation)

| | |
|---|---|
| Example 32 (containing 0.03% L-arginine from rice) | 90.00 mL |
| 2-Methoxyethylamine | |
| (Tokyo Kasei Kogyo) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 35 (1% O-Phosphorylethanolamine + rice preparation containing 0.03% L-arginine + simple preparation)

| | |
|---|---|
| Example 32 (containing 0.03% L-arginine from rice) | 90.00 mL |
| O-Phosphorylethanolamine | |
| (Tokyo Kasei Kogyo) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 36 (1% 2-Ethylaminoethanol + rice preparation containing 0.03% L-arginine + simple preparation)

| | |
|---|---|
| Example 32 (containing 0.03% L-arginine from rice) | 90.00 mL |
| 2-Ethylaminoethanol | |
| (Tokyo Kasei Kogyo) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 37 (1% Diethanolamine + rice preparation containing 0.03% L-arginine + simple preparation)

| | |
|---|---|
| Example 32 (containing 0.03% L-arginine from rice) | 90.00 mL |
| Diethanolamine | |
| (Mitsui Toatsu Chemicals) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 38 (1% 2-Dimethylaminoethanol + rice preparation containing 0.03% L-arginine + simple preparation)

| | |
|---|---|
| Example 32 (containing 0.03% L-arginine from rice) | 90.00 mL |
| 2-Dimethylaminoethanol | |
| (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 39 (1% Choline + rice preparation containing 0.03% L-arginine + simple preparation)

| | |
|---|---|
| Example 32 (containing 0.03% L-arginine from rice) | 90.00 mL |
| Choline (Nakarai Tesuku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 40 (1% 2-Amino-2-hydroxymethyl-1,3-propanediol + rice preparation containing 0.03% L-arginine + simple preparation)

| | |
|---|---|
| Example 32 (containing 0.03% L-arginine from rice) | 90.00 mL |
| 2-Amino-hydroxymethyl-1,3-propanediol | |
| (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 41 (1% Noradrenaline + rice preparation containing 0.03% L-arqinine + simple preparation)

| | |
|---|---|
| Example 32 (containing 0.03% L-arginine from rice) | 90.00 mL |
| Noradrenaline (Nakarai Tesuku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 42 (1% Phenethylamine+ rice preparation containing 0.03% L-arginine + simple preparation)

| | |
|---|---|
| Example 32 (containing 0.03% L-arginine from rice) | 90.00 mL |
| Phenethylamine (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 43 (1% Ethylenediamine + rice preparation containing 0.03% L-arginine + simple preparation)

| | |
|---|---|
| Example 32 (containing 0.03% L-arginine from rice) | 90.00 mL |
| Ethylenediamine (Nakarai Tesuku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 44 (1% Taurine + rice preparation containing 0.03% L-arginine + simple preparation)

| | |
|---|---|
| Example 32 (containing 0.03% L-arginine from rice) | 90.00 mL |
| Taurine (Nakarai Tesuku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 45 (1% Phosphatidylethanolamine + rice preparation containing 0.03% L-arginine + simple preparation)

| | |
|---|---|
| Example 32 (containing 0.03% L-arginine from rice) | 90.00 mL |
| Phosphatidylethanolamine | |
| (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 46 (1% N-(2-Hydroxyethyl)acetamide + rice preparation containing 0.03% L-arginine + simple preparation)

| | |
|---|---|
| Example 32 (containing 0.03% L-arginine from rice) | 90.00 mL |
| N-(2-Hydroxyethyl)acetamide(Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 47 (1% 2-(Methylamino)ethanol + rice preparation containing 0.03% L-arginine + simple preparation)

| | |
|---|---|
| Example 32 (containing 0.03% L-arginine from rice) | 90.00 mL |
| 2-(Metylamino)ethanol (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 48 (1% 2-2-Anilinoethanol + rice preparation containing 0.03% L-arginine + simple preparation)

| | |
|---|---|
| Example 32 (containing 0.03% L-arginine from rice) | 90.00 mL |
| 2-Anilinoethanol (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 49 (1% 2-(Benzylamino)ethanol + rice preparation containing 0.03% L-arginine + simple preparation)

| | |
|---|---|
| Example 32 (containing 0.03% L-arginine from rice) | 90.00 mL |
| 2-(Benzylamino)ethanol (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 50 (1% 3-Amino-1-propanol + rice preparation containing 0.03% L-arginine + simple preparation)

| | |
|---|---|
| Example 32 (containing 0.03% L-arginine from rice) | 90.00 mL |
| 3-Amino-1-propanol (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 51 (1% 2-Amino-1-butanol + rice preparation containing 0.03% L-arginine + simple preparation)

| | |
|---|---|
| Example 32 (containing 0.03% L-arginine from rice) | 90.00 mL |
| 2-Amino-1-butanol (Kanto Kagaku) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 52 (1% Putrescine + rice preparation containing 0.03% L-arginine + simple preparation)

| | |
|---|---|
| Example 32 (containing 0.03% L-arginine from rice) | 90.00 mL |
| Putrescine (Sigma Chemical) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 53 (1% DL-Pyroglutamine acid + rice preparation containing 0.03% L-arginine + simple preparation)

| | |
|---|---|
| Example 32 (containing 0.03% L-arginine from rice) | 90.00 mL |
| DL-Pyroglutamic acid (Tokyo Kasei Kogyo) | 0.90 g |
| 95% Ethanol | 2.00 mL |
| Parabene | 0.18 g |
| Purified soy bean lecithin | 0.05 g |

Make up a final amount of 100.00 g by addition of purified water.

### Example 54 (1% Triethanolamine + rice preparation containing 0.03% L-arginine + simple preparation)

| | |
|---|---|
| Example 32 (containing 0.03% L-arginine from rice) | 90.00 mL |
| Triethanolamine | |
| (Mitsui Toatsu Chemicals) | 0.90 g |
| 95% Ethanol | 2.00 mL |
| Parabene | 0.18 g |
| Purified soy bean lecithin | 0.05 g |

Make up a final amount of 100.00 g by addition of purified water.

### Comparative Example 1 (simple preparation)

| | |
|---|---|
| 95% Ethanol | 2.00 mL |
| Parabene | 0.18 g |
| Purified soy bean lecithin | 0.05 g |

Make up a final amount of 100.00 g by addition of purified water.

### Comparative Example 2 (Hyaluronic acid + simple preparation)

| | |
|---|---|
| Sodium hyaluronate | 1.00 g |
| 95% Ethanol | 2.00 mL |
| Parabene | 0.18 g |
| Purified soy bean lecithin | 0.05 g |

Make up a final amount of 100.00 g by addition of purified water.

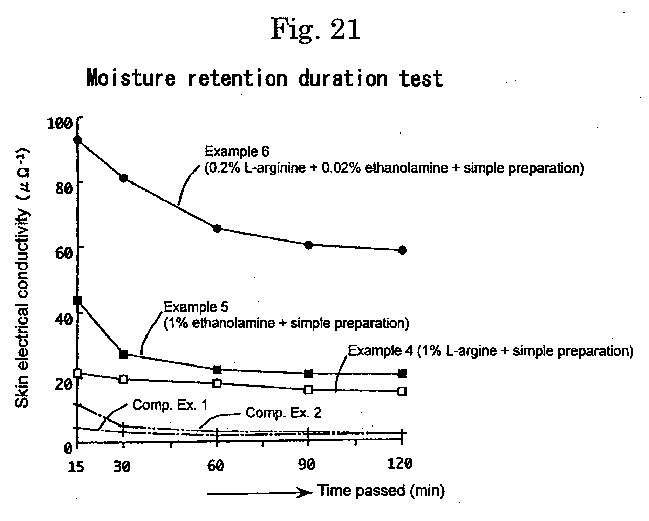

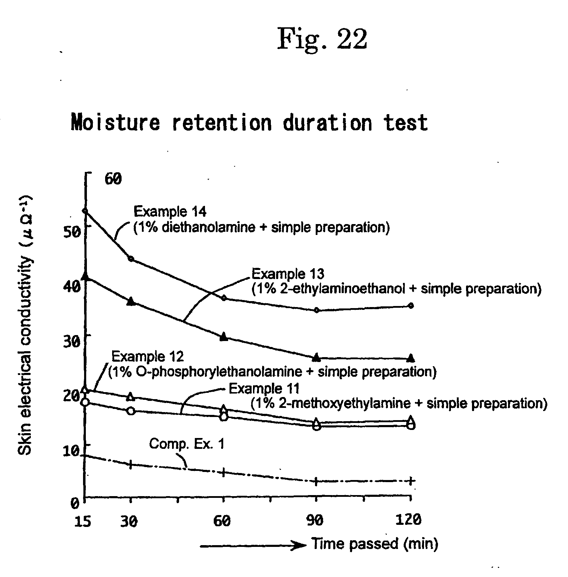

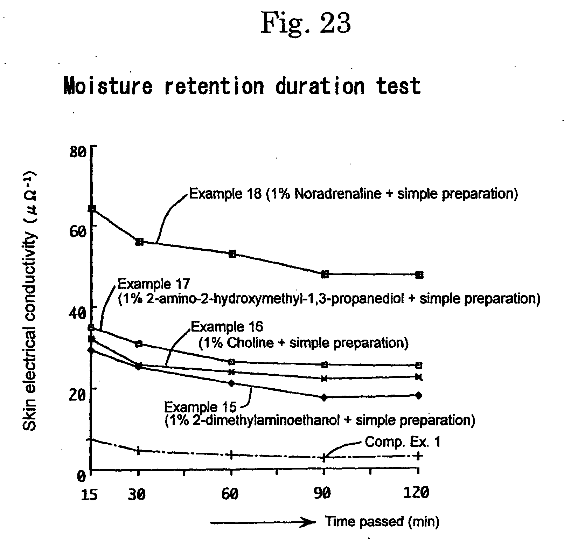

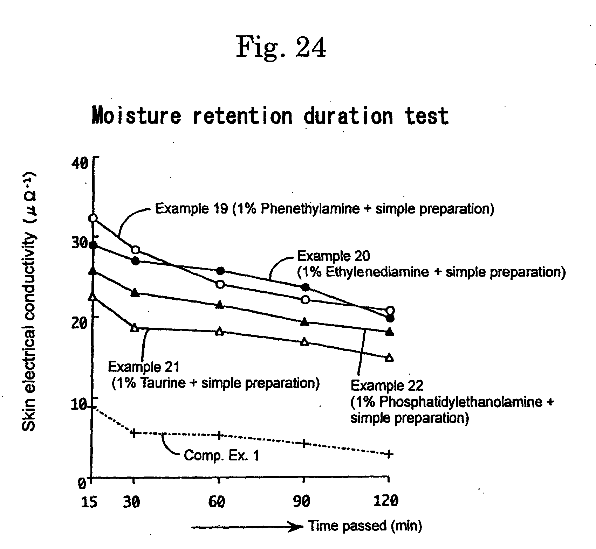

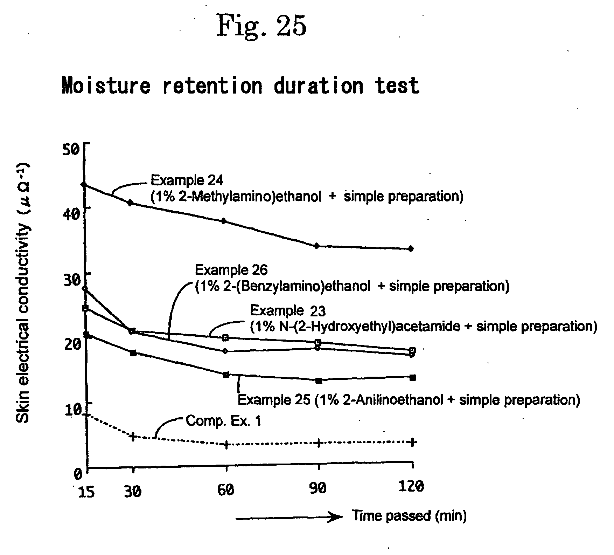

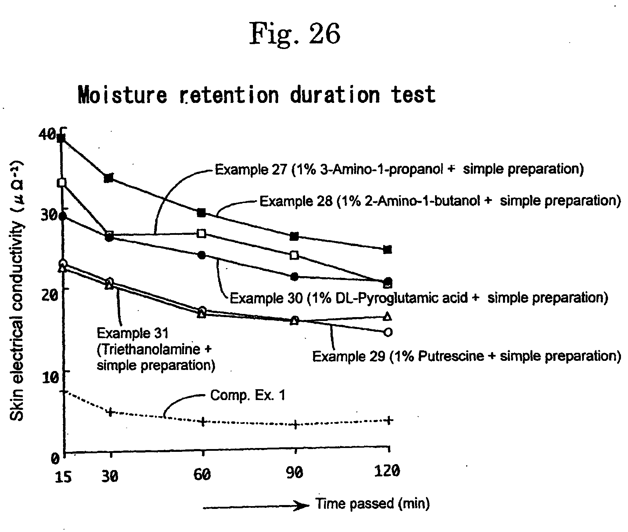

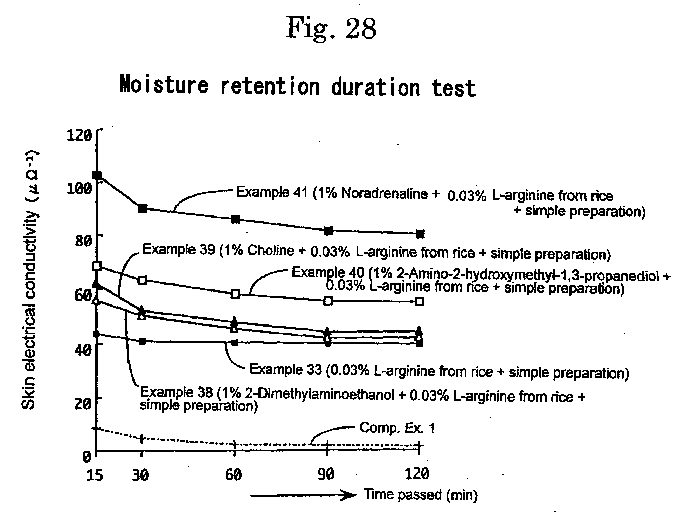

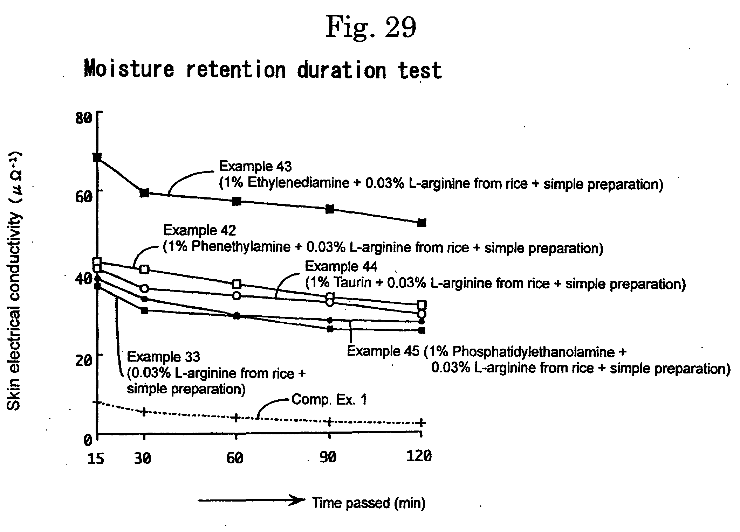

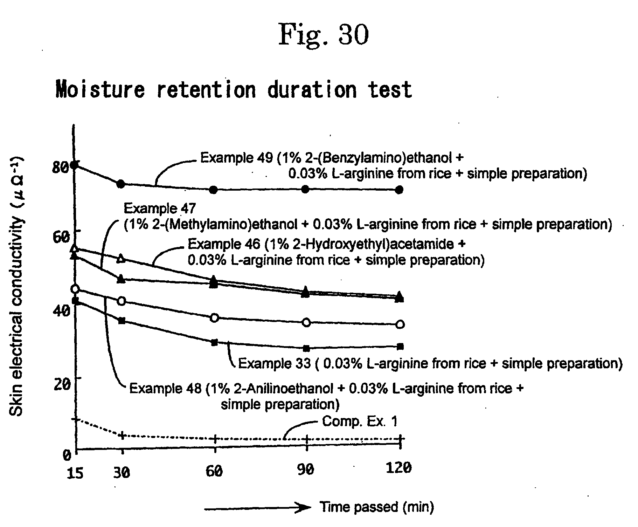

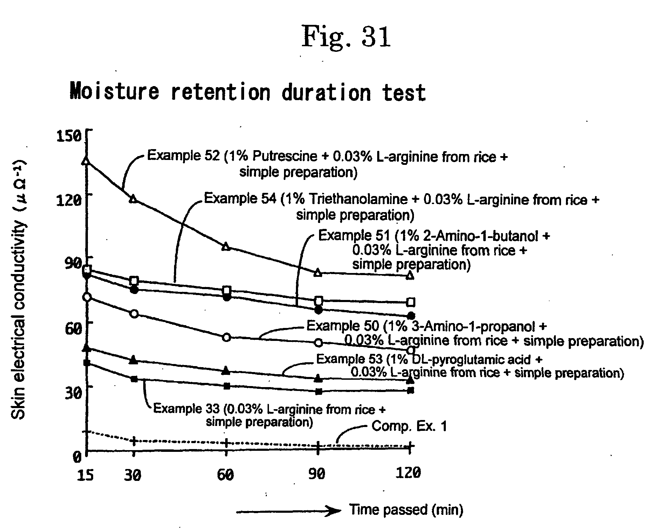

The results of the moisture retention duration test are as shown in Figs. 21 through 31. With respect to atopic skin, moisture retention effects were remarkable 15 minutes after application, and moisture retention continued beyond 30 minutes for 2 hours. Although continuation of moisture retention was observed with either L-arginine or ethanolamine alone, when two kinds of substances were present, moisture retention duration was enhanced more than when either substance was used alone even at lower concentrations (see Example 6 in Fig. 21).

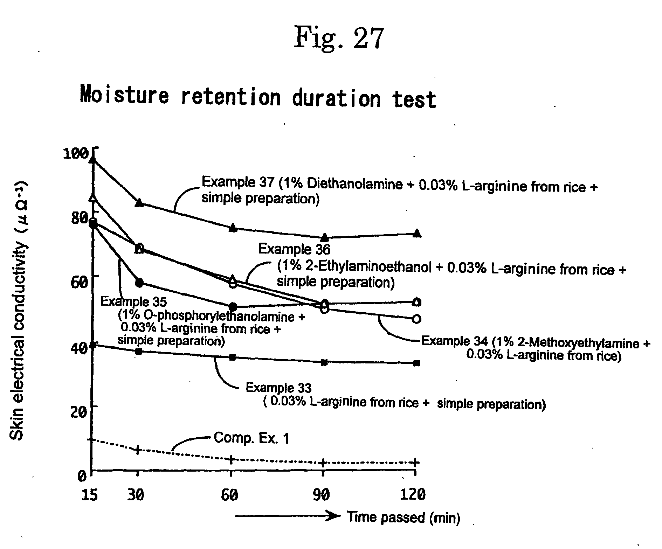

Although Examples 34 through 54 (referred to as the "former") are mixtures containing 0.03% L-arginine in Examples 11 through 31 (referred to as the "latter"), respectively, the former demonstrated higher moisture retention duration than the latter (see Figs. 27 through 31).

### Test Example 10 (Test for conditioning the skin's barrier mechanism and function, particularly tests for conditioning the corneal layer of epidermis, and preventing, preventing exacerbation of and treating atopic dermatitis)

A moisture retention ability test was performed on atopic skin.
Panelists: 4 persons with atopic skin
Measurement Method: Same as measurement method of Test Example 3
Test Apparatus: Same as test apparatus of Test Example 2

The samples were as shown below.

### Example 4 (1% L-arginine simple preparation)

### Example 5 (1% Ethanolamine simple preparation)

### Example 6 (0.2% L-arginine + 0.02% Ethanolamine + simple preparation)

### Example 11 (1% 2-Methoxyethylamine simple preparation)

### Example 14 (1% Diethanolamine simple preparation)

### Example 16 (1% Choline simple preparation)

### Example 17 (1% 2-Amino-hydroxymethyl-1,3-propanediol simple preparation)

### Example 18 (1% Noradrenaline simple preparation)

### Example 34 (1% 2-Methoxyethylamine + rice preparation containing 0.03% L-arginine + simple preparation)

### Example 37 (1% Diethanolamine + rice preparation containing 0.03% L-arginine + simple preparation)

### Example 39 (1% Choline + rice preparation containing 0.03% L-arginine + simple preparation)

### Example 40 (1% 2-Amino-2-hydroxymethyl-1,3-propanediol + rice preparation containing 0.03% L-arginine + simple preparation)

### Example 41 (1% Noradrenaline + rice preparation containing 0.03% L-arginine + simple preparation)

### Comparative Example 1 (simple preparation)

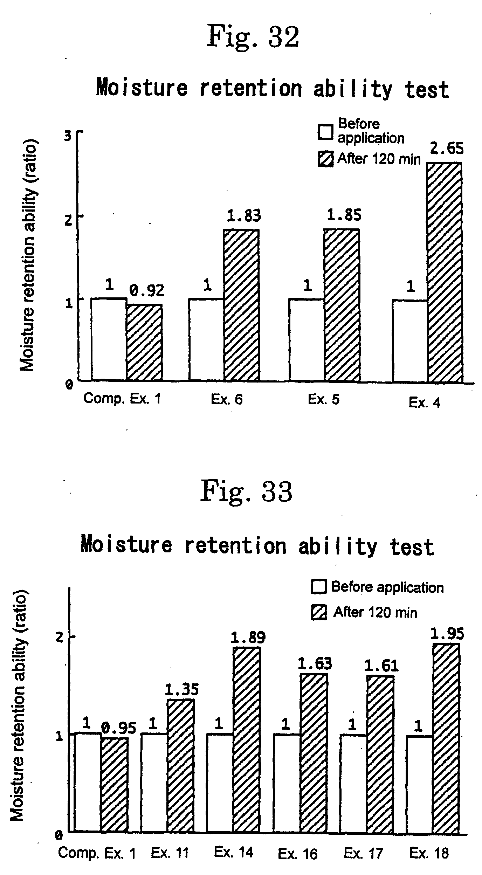

The results of the moisture retention ability test are as shown in Figs. 32 through 34.

Although effects that increased moisture retention ability for atopic skin were not observed at all for Comparative Example 1, the moisture retention ability 2 hours after applying the samples of the above-mentioned Examples of the present invention increase significantly as compared with before application.

In Fig. 32, although moisture retention ability increased with either L-arginine alone (Example 4) or ethanolamine alone (Example 5), in the case both substances were present (Example 6), moisture retention ability was increased more than when either substance was used alone even at lower concentrations.

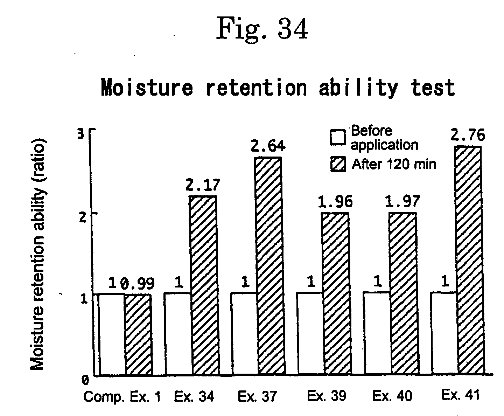

Although Examples 34 through 41 (referred to as the "former") are mixtures of rice preparations containing 0.03% L-arginine with Examples 11 through 18 (referred to as the "latter"), respectively, the former demonstrated higher moisture retention ability than the latter (Fig. 34).

In this manner, the samples of the present invention increased the skin's barrier function by acting on the corneal layer, and acted on epidermal keratocytes not present in the corneal layer to produce a corneal layer having a high barrier function.

### Test Example 11 (Test for conditioning the skin's barrier mechanism and function, particularly tests for conditioning the corneal layer of epidermis, and preventing, preventing exacerbation of and treating atopic dermatitis)

The amount of moisture loss from the skin (transepidermal moisture evaporation volume) was measured to confirm barrier function improvement effects.
Panelists: 4 persons with atopic skin
Test Method: Each sample was applied to the side of the forearm of the panelists (approx. 0.3 x 0.3 cm) followed by measurement of transepidermal moisture evaporation volume at 60 and 120 minutes after application.

Measurement Method:
(1) The test site is washed with soap.
(2) The test site is exposed in a constant temperature and constant humidity room at a temperature of 20°C and humidity of 50%, and the skin is allowed to reach a steady state by allowing the panelists to rest quietly starting 60 minutes before measurement.
(3) Transepidermal water loss (TWEL) at the test site is measured for about 1 minute (the rate of moisture evaporation at the test site is measured as TEWL (g/m²·h) automatically by software computation by contacting a cylindrical probe of the TEWAMETER TM210 perpendicular to the test site).

Test Apparatus:
TEWAMETER TM210 (Nippon Eurotech)
TEWAMETER Software Ver. 1.1 (Nippon Eurotech)
Samples: Same as the samples used in Test Example 10

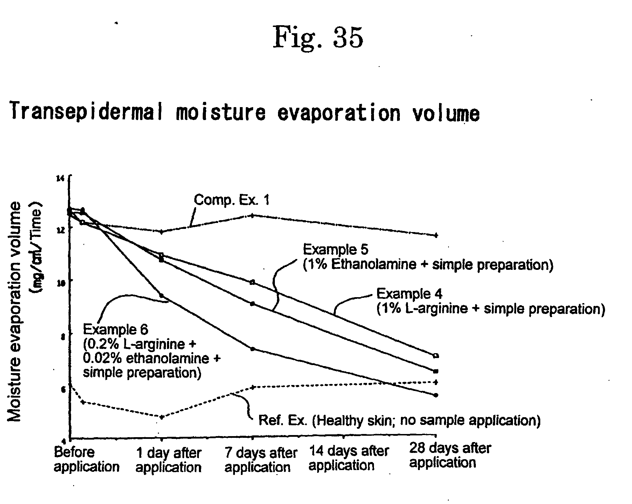

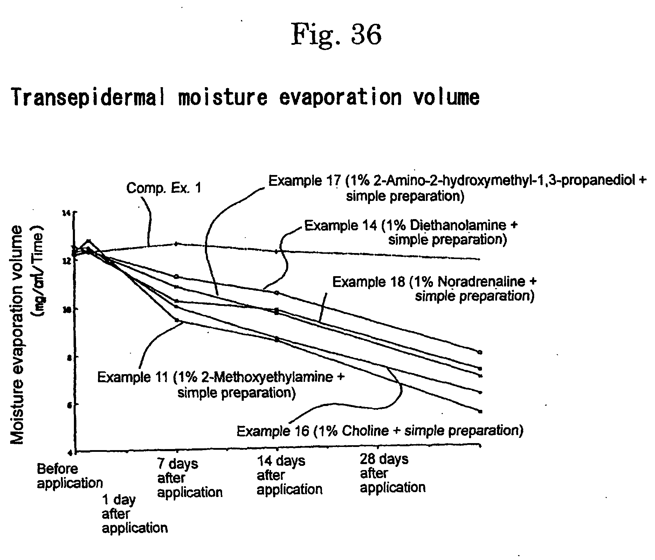

The test results for transepidermal moisture evaporation volume are as shown in Figs. 35 through 37.

The amount of moisture loss is greater in atopic skin prior to application of the samples of the present invention as compared with healthy skin due to a decrease in the skin's barrier function. The amount of moisture loss was decreased nearly to the level of healthy skin following application of the samples of the present invention to atopic skin for 4 weeks, and the skin's barrier mechanism and function were determined to have been improved.

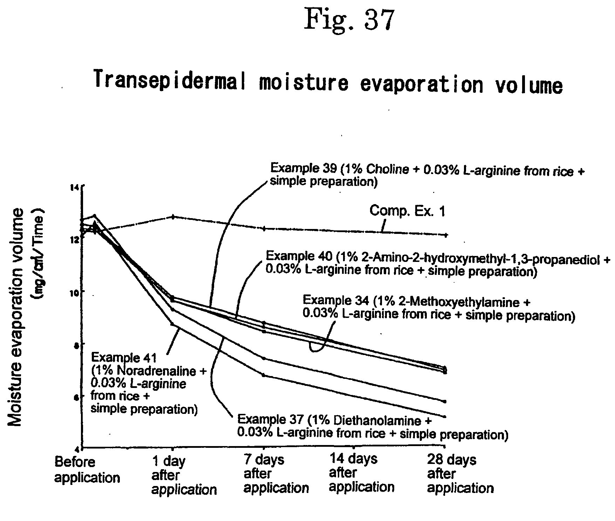

Although Examples 34 through 41 (referred to as the "former") are mixtures of rice preparations containing 0.03% L-arginine with Examples 11 through 18 (referred to as the "latter"), respectively, the former demonstrated greater effects that reduced the amount of moisture loss than the latter (Fig. 37).

In this manner, impairment of the skin's barrier mechanism and function was improved, and internal moisture loss was inhibited by applying the samples of the present invention to atopic skin.

### Test Example 12 (Test for conditioning the skin's barrier mechanism and function, particularly tests for conditioning the corneal layer of epidermis, and preventing, preventing exacerbation of and treating atopic dermatitis)

An allergic reaction inhibition test was conducted in house dust-sensitized model animals (guinea pigs).

### Experimental Animals: Guinea pigs, 6

### Test Method:

(1) House dust extract and adjuvant were mixed and injected subcutaneously into the guinea pigs to sensitize.
(2) After sensitization was established, the abdomens of the guinea pigs were shaved to produce chapped skin.
(3) The samples were applied to the site where chapped skin was produced.
(4) House dust extract was applied to the sample application site.
(5) Skin reaction was evaluated for 1-5 days after step (4).

Evaluation of the induced skin reaction (dermatitis) was scored based on the following standards.
0: No reaction
1: Mild erythema
2: Moderate erythema
3: Serious erythema
4: Serious erythema accompanied by edema

The samples were as shown below.

### Example 55 (Simple preparation containing 40% Example 3)

| | |
|---|---|
| Example 3 (containing 0.2% L-arginine and 0.02% ethanolamine from rice) | 40.00 mL |
| 95% Ethanol | 2.00 mL |
| Parabene | 0.18 g |
| Purified soy bean lecithin | 0.05 g |

Make up a final amount of 100.00 g by addition of purified water.

### Comparative Example 1 (simple preparation)

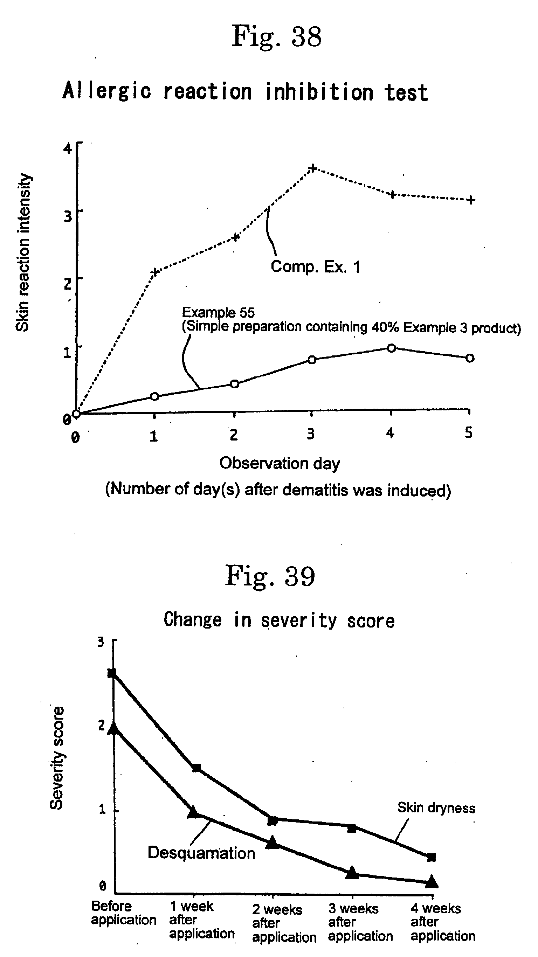

The results of the allergic reaction inhibition test in house dust-sensitized model animals (guinea pigs) are shown in Fig. 38.

When a sample of the present invention was applied to house dust-sensitized guinea pig skin in which chapped skin had been produced artificially followed by reapplication of house dust, the degree of house dust extract-induced dermatitis was inhibited over the course of 5 days after application.

In this manner, skin in which impairment of the skin's barrier mechanism and function was improved by application of a sample of the present invention was able to prevent infiltration of antigen from the outside and inhibit dermatitis.

### Test Example 13 (Tests for conditioning the skin's barrier mechanism and function, and for preventing, preventing exacerbation of and treating atopic dermatitis)

A clinical test was conducted on atopic skin of patients with atopic dermatitis.
Panelists: 12 patients with atopic dermatitis

### Test Sites:

The test sites consisted of sites having symptoms suitable for evaluation that enabled comparison of a site using Example 8 and a site using Comparative Example 5 either to the left and right or above and below.

### External Application Method:

Simple application at each site separately for Example 8 and Comparative Example 5 twice per day (morning and evening).

### Application Period: 4 weeks

### Evaluation Items:

Evaluation items consisted of the main symptoms of atopic skin.
(1) Skin dryness
(2) Scaling
(3) Itchiness

### Evaluation Method:

The results of the site where Example 8 was applied were evaluated for the evaluation items according to the following four levels of a severity score as determined by visual examination.
3: Advanced symptoms
2: Moderate symptoms
1: Mild symptoms
0: No symptoms or symptoms disappeared

In addition, improvement (usefulness) of effects as compared with Comparative Example 5 was evaluated each week according to the following four levels:
Extremely useful
Useful
Somewhat useful
Not useful
Finally, the usefulness of the present invention was evaluated in terms of the overall usefulness throughout the usage period.

The samples were as shown below.

### Example 8 (Example 3 + cream preparation)

| | |
|---|---|
| Example 3 (containing 0.2% L-arginine and 0.02% ethanolamine from rice) | 40.00 mL |
| Dipotassium glycyrrhetinate | 0.10 g |
| 1,3-Butyleneglycol | 6.00 g |
| Concentrated glycerin | 6.00 g |
| Methylpolysiloxane | 6.00 g |
| Stearic acid | 3.00 g |
| Cetanol | 3.00 g |
| Cetyl 2-ethylhexanoate | 6.00 g |
| Squalene | 6.00 g |
| Sucrose fatty acid ester | 3.00 g |
| dl-α-Tocopherol acetate | 0.30 g |
| Sodium casein | 1.50 g |
| Disodium edetate | 0.03 g |
| Parabene | 0.30 g |

Make up a final amount of 100.00 g by addition of purified water.

### Comparative Example 5 (Cream preparation)

| | |
|---|---|
| Dipotassium glycyrrhetinate | 0.10 g |
| 1,3-Butyleneglycol | 6.00 g |
| Concentrated glycerin | 6.00 g |
| Methylpolysiloxane | 6.00 g |
| Stearic acid | 3.00 g |
| Cetanol | 3.00 g |
| Cetyl 2-ethylhexanoate | 6.00 g |
| Squalene | 6.00 g |
| Sucrose fatty acid ester | 3.00 g |
| dl-α-Tocopherol acetate | 0.30 g |
| Sodium casein | 1.50 g |
| Disodium edetate | 0.03 g |
| Parabene | 0.30 g |

Make up a final amount of 100.00 g by addition of purified water.

### Test Results:

When a sample of the present invention and Comparative Example 5 were respectively used on skin susceptible to the induction of dermatitis (atopic skin) located near to the affected area of atopic dermatitis patients, in contrast to Comparative Example 5 being completely ineffective, the present invention demonstrated a high degree of usefulness.

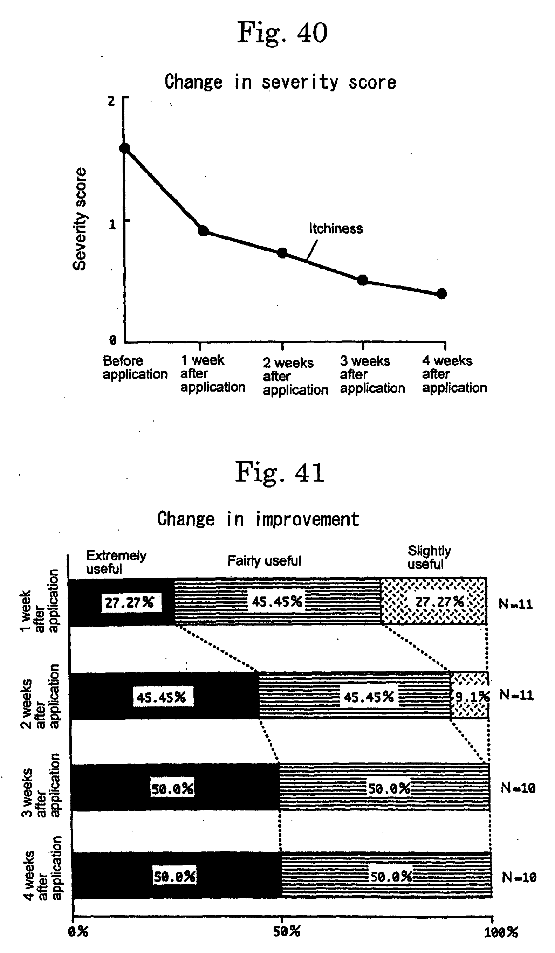

Figs. 39 through 42 show the changes in severity scores of skin dryness, scaling and itchiness. According to these results, the present invention alleviated skin symptoms such as skin dryness, scaling and itchiness associated with atopic dermatitis, and was observed to demonstrate a high degree of usefulness against each of these symptoms. There were no adverse side effects observed and a high degree of safety was observed.

Since the present invention has remarkable effects against itchiness, the vicious circle of itchiness leading to scratching, scratching leading to increased itchiness, and further scratching leading to exacerbation of atopic dermatitis can be terminated, thereby making it possible to prevent the onset and exacerbation of atopic dermatitis. In addition, as a result of being freed from itchiness, the present invention also has effects on the mental state of atopic dermatitis patients.

In this manner, the present invention is able to improve skin symptoms of skin dryness, scaling and itchiness observed in atopic skin, thereby being able to prevent the onset and exacerbation of atopic dermatitis, by restoring the skin's barrier mechanism and function through conditioning of the skin.

### Test Example 14 (Tests for conditioning the skin's barrier mechanism and function, and for preventing, preventing exacerbation of and treating atopic dermatitis)

A clinical test was conducted on the affected skin of atopic dermatitis patients to observe the therapeutic effects on atopic dermatitis as a result of skin conditioning and restoration of the skin's barrier mechanism and function.
Panelists: 7 patients with atopic dermatitis
Samples: Sample as in the case of Test Example 13.

### Example 8 (Example 3 + cream preparation)

### Comparative Example 5 (Cream preparation)

### Test Sites:

The test sites consisted of sites having symptoms suitable for evaluation that enabled comparison of a site using Example 8 and a site using Comparative Example 5 either to the left and right or above and below.

### External Application Method:

Simple application at each site separately for Example 8 and Comparative Example 5 twice per day (morning and evening).

### Application Period: 4 weeks

### Evaluation Items:

### Evaluation items consisted of:

(1) Itchiness
(2) Scratched scar
(3) Erythema
(4) Lichenification

### Evaluation Method:

The results of the site where Example 8 was applied were evaluated for the evaluation items according to the following four levels of a severity score as determined by visual examination.
3: Advanced symptoms
2: Moderate symptoms
1: Mild symptoms
0: No symptoms or symptoms disappeared

In addition, improvement (usefulness) of effects as compared with Comparative Example 5 was evaluated each week according to the following four levels:
Extremely useful
Useful
Somewhat useful
Not useful
Finally, the usefulness of the present invention was evaluated in terms of the overall usefulness throughout the usage period.

### Test Results:

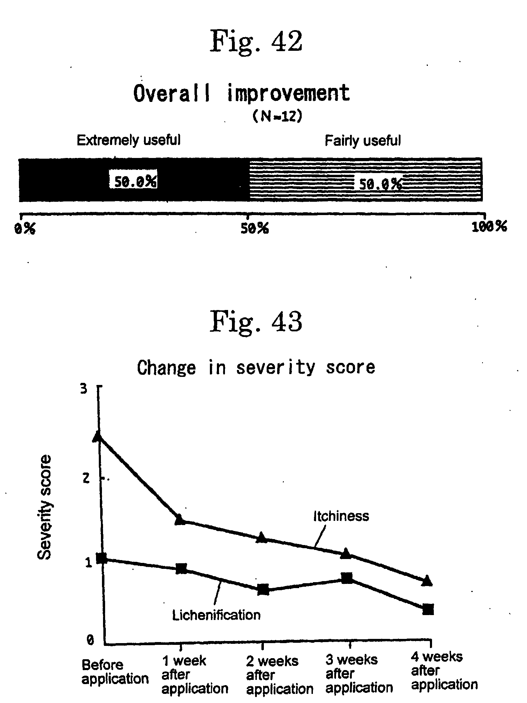

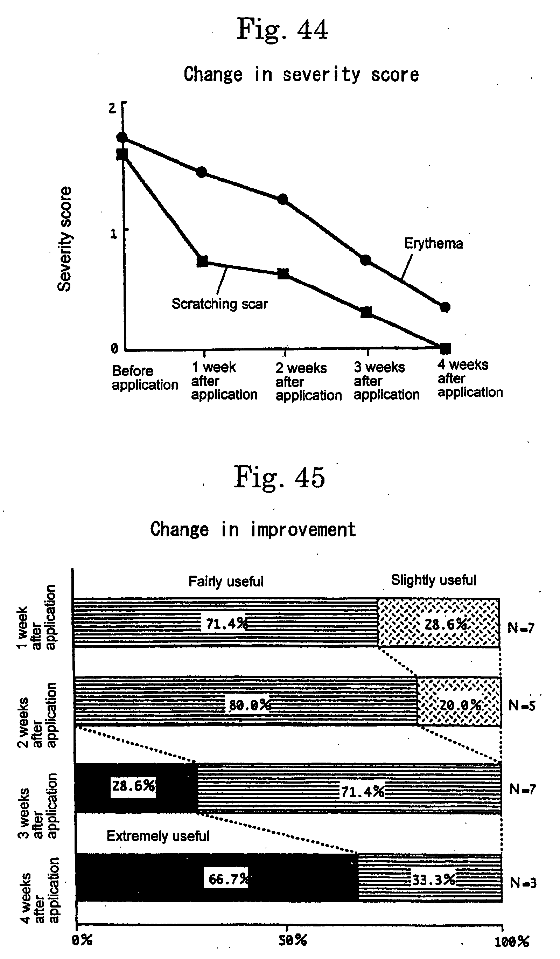

When a sample of the present invention and Comparative Example 5 were respectively used on atopic dermatitis patients, in contrast to Comparative Example 5 being completely ineffective, the present invention demonstrated a high degree of usefulness as shown in Figs. 43 through 46.

Figs. 43 through 46 show the changes in severity scores of itchiness, scratched scars, erythema and lichenification at the site of use of Example 8 of the present invention. According to these results, the present invention alleviated skin symptoms such as itchiness, scratched scars, erythema and lichenification associated with atopic dermatitis, and was observed to demonstrate a high degree of usefulness against each of these symptoms. There were no adverse side effects, rebound phenomena were not observed following discontinuation of use, and there were no cases of recurrence.

Since the present invention has remarkable effects against itchiness, the vicious circle of itchiness leading to scratching and scratching leading to exacerbation of atopic dermatitis can be terminated, and it is possible to prevent the onset and exacerbation of atopic dermatitis. In addition, as a result of being freed from itchiness, the present invention also has effects on the mental state of atopic dermatitis patients.

In this manner, the present invention is able to improve skin symptoms of itchiness, scratched scars, erythema and lichenification observed in atopic dermatitis, thereby being able to heal this disease, through conditioning of the skin.

### Test Example 15 (Tests for conditioning the skin's barrier mechanism and function, and for preventing, preventing exacerbation of and treating atopic dermatitis)

A clinical test was conducted on the affected skin of atopic dermatitis patients to observe the therapeutic effects on atopic dermatitis as a result of skin conditioning and restoration of the skin's barrier mechanism and function.

### Panelists: 5 patients with atopic dermatitis

### Samples:

### Example 56 (1% Ethanolamine + cream preparation)

| | |
|---|---|
| 1% aqueous solution of Ethanolamine | |
| (Nakarai Tesuku) | 1.00 g |
| Dipotassium glycyrrhetinate | 0.10 g |
| 1,3-Butyleneglycol | 6.00 g |
| Concentrated glycerin | 6.00 g |
| Methylpolysiloxane | 6.00 g |
| Stearic acid | 3.00 g |
| Cetanol | 3.00 g |
| Cetyl 2-ethylhexanoate | 6.00 g |
| Squalene | 6.00 g |
| Sucrose fatty acid ester | 3.00 g |
| dl-α-Tocopherol acetate | 0.30 g |
| Sodium casein | 1.50 g |
| Disodium edetate | 0.03 g |
| Parabene | 0.30 g |

Make up a final amount of 100.00 g by addition of purified water.

### Comparative Example 5 (Cream preparation)

### Test Sites:

The test sites consisted of sites having symptoms suitable for evaluation that enabled comparison of a site using Example 56 and a site using Comparative Example 5 either to the left and right or above and below.

### External Application Method:

Simple application at each site separately for Example 56 and Comparative Example 5 twice per day (morning and evening).

### Application Period: 4 weeks

### Evaluation Items: Same as Test Example 13

### Evaluation Method: Same as Test Example 13

### Test Results:

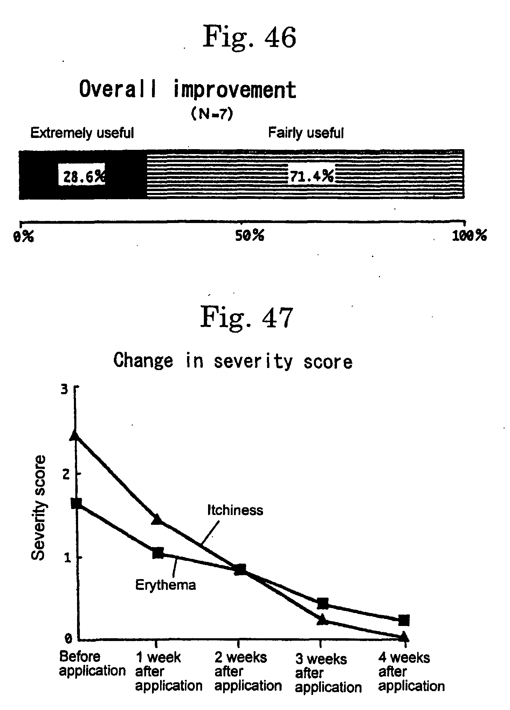

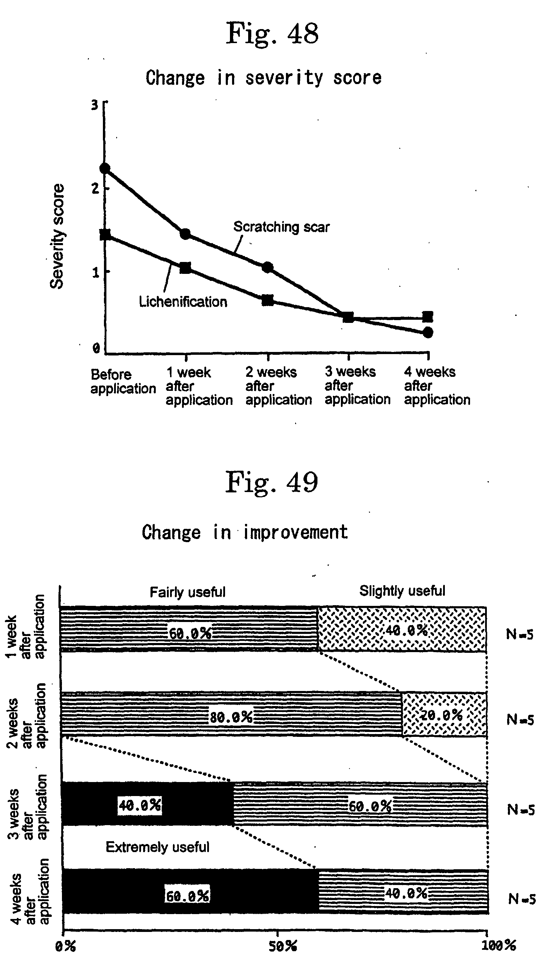

When Example 56 of the present invention and Comparative Example 5 were respectively used on atopic dermatitis patients, in contrast to Comparative Example 5 being completely ineffective, Example 56 of the present invention demonstrated a high degree of usefulness as shown in Figs. 47 through 50.

Figs. 47 through 50 show the changes in severity scores of itchiness, scratched scars, erythema and lichenification at the site of use of Example 56 of the present invention. According to these results, Example 56 of the present invention alleviated skin symptoms such as itchiness, scratched scars, erythema and lichenification associated with atopic dermatitis, and was observed to demonstrate a high degree of usefulness against each of these symptoms.There were no adverse side effects, rebound phenomena were not observed following discontinuation of use, and there were no cases of recurrence.

Since the present invention has remarkable effects against itchiness, the vicious circle of itchiness leading to scratching and scratching leading to exacerbation of atopic dermatitis can be terminated, and it is possible to prevent the onset and exacerbation of atopic dermatitis. In addition, as a result of being freed from itchiness, the present invention also has effects on the mental state of atopic dermatitis patients.

In this manner, the present invention is able to improve skin symptoms of itchiness, scratched scars, erythema and lichenification observed in atopic dermatitis, thereby being able to heal this disease, through conditioning of the skin.

### Test Example 16 (Test for conditioning the skin's barrier mechanism and function, particularly tests for conditioning the corneal layer of the epidermis, for conditioning the epidermal keratocytes, for promoting the production of the healthy corneal layer of the epidermis, and for normalizing cell differentiation)

The change in moisture retention ability was measured when samples were applied on persons with chapped skin and atopic skin for a long time to observe their effects on the entire epidermis.

### Samples:

### Example 6 (L-arginine + ethanolamine + simple preparation)

### Example 8 (L-arginine + ethanolamine + cream preparation)

### Comparative Example 1 (simple preparation)

### Comparative Example 2 (Hyaluronic acid + simple preparation)

Samples of Example 6, Comparative Example 1 and Comparative Example 2 were applied to persons with chapped skin and a sample of Example 8 was applied to person with atopic skin.
Panelists: 9 volunteers with chapped skin
   3 volunteers with atopic skin
Test Sites: Sides of upper arm (samples of Examples and of Comparative Examples were applied separately to the left side and the right side, respectively

External Application Method: Simple application twice per day (morning and evening).

### Application Period: 4 weeks

Measurement Method: Moisture retention ability before application, 2 weeks after application, 4 weeks after application and 2 weeks after discontinuation of application of samples were measured in accordance with the method of Test Example 3.
(1) Same as Test Example 3
(2) Same as Test Example 3
(3) Same as Test Example 3
(4) Same as Test Example 3

The method to determine moisture retention ability is the same as Test Example 3. It should be noted that, moisture retention ability (ratio) was expressed as the ratio obtained when the moisture retention ability before application of a sample (%) is given a value of 1.

Test Apparatus: Same as Test Example 2.

### Test Results:

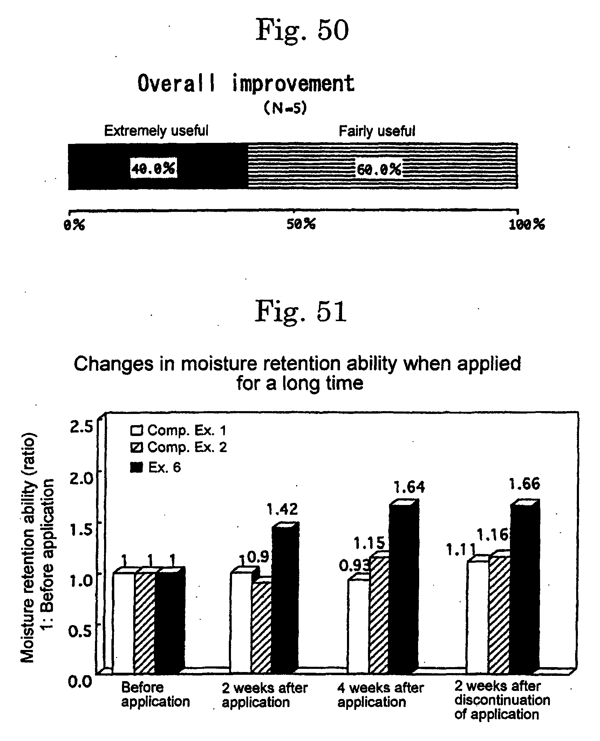

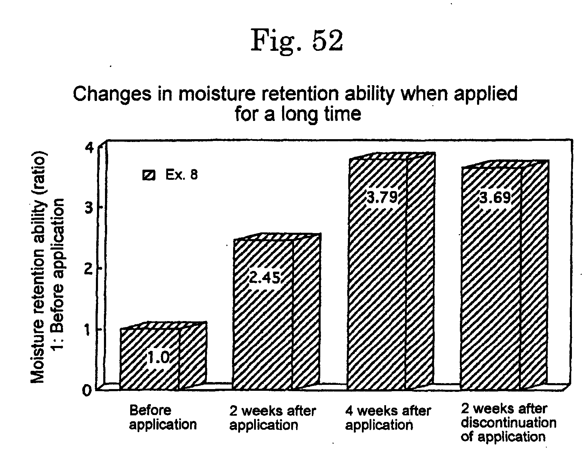

The results of tests on persons with chapped skin are shown in Fig. 51 and the results of tests on persons with atopic skin are shown in Fig. 52. The present invention when applied for a long time increased its moisture retention ability with a lapse of time during the application period. Moreover, the enhanced moisture retention ability when applied for a long time was sustained 2 weeks after discontinuation of application. On the other hand, in Comparative Example 1 and Comparative Example 2, no effects were observed even during the period of application.

With enhanced effects in the moisture retention ability exhibited 2 weeks after discontinuation of application, the present invention proved to condition not only the corneal layer of epidermis but also the epidermal keratocytes, to promote the production of a healthy corneal layer of the epidermis and to normalize cell differentiation. It was demonstrated that the present invention conditions the entire epidermis.

### Test Example 17 (Test for effect on conditioning the skin's barrier mechanism and function, particularly for conditioning the corneal layer of the epidermis)

A moisture retention duration test was conducted on persons with chapped skin.
Panelists: 7 volunteers with chapped skin
Test Method: Same as Test Example 2
Measurement Method: Same as Text Example 2
Test Apparatus: Same as Test Example 2

The samples were as shown below.

### Example 55 (1% Aqueous ammonia + simple preparation)

| | |
|---|---|
| 28% Aqueous ammonia | |
| (Wako Pure Chemical Ind. Ltd.) | 3.57 mL |
| 95% Ethanol | 2.00 mL |
| Parabene | 0.18 g |
| Purified soy bean lecithin | 0.05 g |

Make up a final amount of 100.00 g by addition of purified water.

### Example 56 (Aqueous ammonia + L-arginine + simple preparation)

| | |
|---|---|
| 28% Aqueous ammonia | |
| (Wako Pure Chemical Ind. Ltd.) | 3.57 mL |
| L-arginine (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 57 (Aqueous ammonia + ethanolamine + simple preparation)

| | |
|---|---|
| 28% Aqueous ammonia | |
| (Wako Pure Chemical Ind. Ltd.) | 3.57 mL |
| Ethanolamine (Nakarai Tesuku) | 1.00 g |
| 95% Ethanol | 2.00 mL |
| Parabene | 0.18 g |
| Purified soy bean lecithin | 0.05 g |

Make up a final amount of 100.00 g by addition of purified water.

### Example 58 (Aqueous ammonia + L-arginine + ethanolamine +simple preparation)

| | |
|---|---|
| 28% Aqueous ammonia | |
| (Wako Pure Chemical Ind. Ltd.) | 0.36 mL |
| L-arginine (Nakarai Tesuku) | 1.00 g |
| Ethanolamine (Nakarai Tesuku) | 0.10 g |
| 95% Ethanol | 2.00 mL |
| Parabene | 0.18 g |
| Purified soy bean lecithin | 0.05 g |

Make up a final amount of 100.00 g by addition of purified water.

### Example 59 (Rice preparation containing 0.03% L-arginine + aqueous ammonia + L-arginine + ethanolamine + simple preparation)

| | |
|---|---|
| Example 32 (containing 0.03% L-arginine) | 90.00 mL |
| 28% Aqueous ammonia | |
| (Wako Pure Chemical Ind. Ltd.) | 0.357 mL |
| L-arginine (Nakarai Tesuku) | 0.10 g |
| Ethanolamine (Nakarai Tesuku) | 0.10 g |
| 95% Ethanol | 2.00 mL |
| Parabene | 0.18 g |
| Purified soy bean lecithin | 0.05 g |

Make up a final amount of 100.00 g by addition of purified water.

### Comparative Example 1

### Test Results:

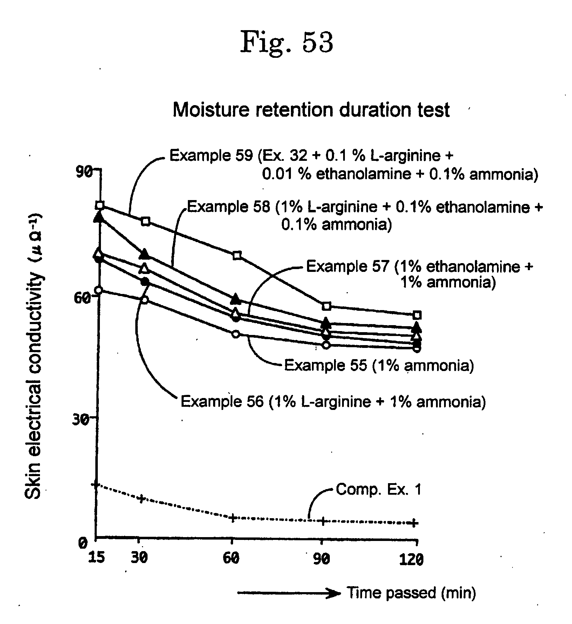

As shown in Fig. 53, Examples 55 to 59 exhibit remarkable moisture retention effects 15 minutes after application and sustain moisture retention over 2 hours after 30 minutes. While this moisture retention duration can be observed with ammonium ions alone, presence of L-arginine or ethanolamine exhibits further remarkable effects. Moreover, when three of ammonium ions, L-arginine and ethanolamine are present, effects will be further enhanced. When a rice preparation is present, even small amount of ammonium ions, L-arginine and ethanolamine will exhibits remarkable effects.

### Test Example 18 (Test for effect on conditioning the skin's barrier mechanism and function, particularly for conditioning the corneal layer of the epidermis)

A moisture retention ability test was performed on atopic skin.
Panelists: 4 persons with atopic skin
Test Method: Same as Test Example 3
Measurement Method: Same as Text Example 3
Test Apparatus: Same as Test Example 3

The samples were as shown below.

### Example 55 (1% Aqueous ammonia + simple preparation)

### Example 56 (Aqueous ammonia + L-arginine + simple preparation)

### Example 57 (Aqueous ammonia + ethanolamine + simple preparation)

### Example 58 (Aqueous ammonia + L-arginine + ethanolamine +simple preparation)

### Example 59 (Rice preparation containing 0.03% L-arginine + aqueous ammonia + L-arginine + ethanolamine + simple preparation)

### Comparative Example 1

### Test Results:

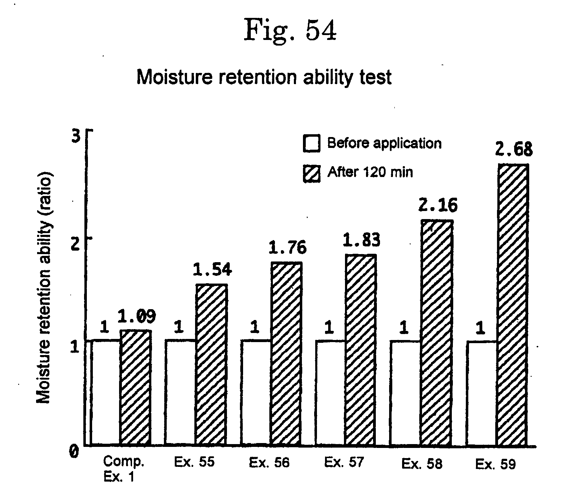

As shown in Fig. 54, all of Examples 55 to 59 increase their moisture retention ability. While this improved moisture retention duration can be observed with the presence of ammonium ions alone, presence of L-arginine or ethanolamine exhibits more remarkable effects. Moreover, when the three of ammonium ions, L-arginine and ethanolamine are present, effects are further enhanced. Furthermore, when a rice preparation is present, even a small amount of ammonium ions, L-arginine and ethanolamine exhibits remarkable effects.

### Test Example 19 (Test for effect on conditioning the skin's barrier mechanism and function, particularly for conditioning the corneal layer of the epidermis, for conditioning the epidermal keratocytes, for promoting the production of the perfect corneal layer of the epidermis, and for normalizing cell differentiation)

The change in moisture retention ability was measured when samples were applied on persons with atopic skin for a long time to observe their effects on the entire epidermis.

### Samples:

### Example 59 (Rice preparation containing 0.03% L-arginine + aqueous ammonia + L-arginine + ethanolamine + simple preparation)

Panelists: 4 persons with atopic skin
Test Sites: Sides of upper arm
External Application Method: Simple application twice per day (morning and evening).
Application Period: 4 weeks
Measurement Method: Same as Text Example 16
Test Apparatus: Same as Test Example 2

### Test Results:

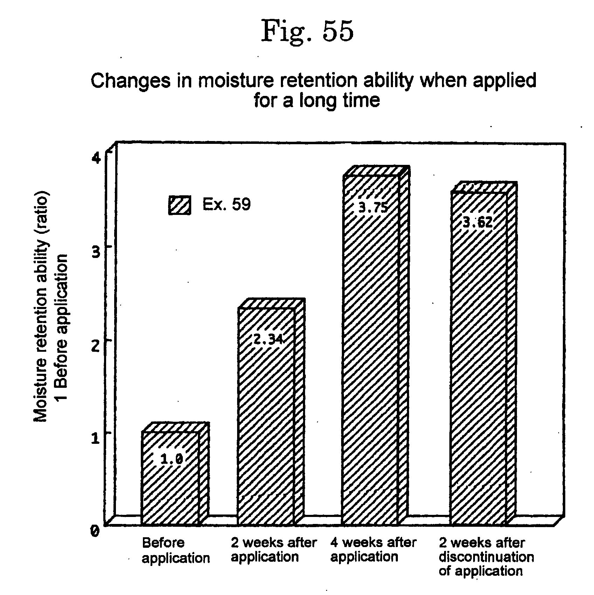

As shown in Fig. 55, the sample according to the present invention when applied for a long time increased moisture retention ability with time during the application period. Moreover, enhanced moisture retention ability when applied for a long time was sustained 2 weeks after discontinuation of application. With enhanced effects of moisture retention ability exhibited 2 weeks after discontinuation of application, a sample according to the present invention proved to condition not only the corneal layer of epidermis but also the epidermal keratocytes, to promote the production of the healthy corneal layer of the epidermis and to normalize cell differentiation. It demonstrated to condition the entire epidermis.

### Industrial Applicability

The present invention relates to a skin conditioner comprising ammonium salts and/or the compound represented by the formula (1): 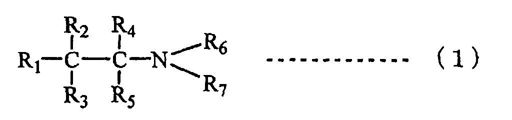 (wherein, the symbols are the same as those defined in the text). Examples of active ingredients of the present invention include L-arginine and ethanolamine. These active ingredients can be acquired as chemical synthesis products, or they may also be acquired in the form of natural substances. Preferable Examples of natural substances include substances containing L-arginine and/or ethanolamine obtained from rice. The skin conditioner as claimed in the present invention demonstrates remarkable effectiveness as an agent for restoring the skin's barrier mechanism and function, as an agent for the prevention and treatment of atopic dermatitis and as a skin moisture retention agent.

## Claims

1. A skin conditioner containing one or more kinds of ingredient selected from the group consisting of an ammonium salt and ions thereof, a compound represented by the formula (1): 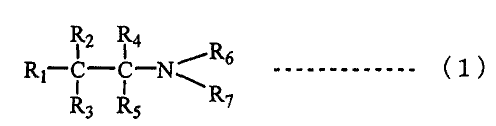 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a carboxyl group, a guanidino group or a lower alkyl group substituted with a guanidino group;
R₄ and R₅ each independently represent a hydrogen atom, a hydroxyl group, a lower alkyl group or an aryl group that may optionally be substituted with a hydroxyl group or an amino group, or a carboxyl group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and ions thereof and pharmaceutically acceptable salts thereof.

2. A skin conditioner according to claim 1 wherein the skin conditioner is an agent for restoration of the skin's barrier mechanism and function.

3. A skin conditioner according to claim 1 wherein the skin conditioner is an agent for the prevention, prevention of exacerbation or treatment of atopic dermatitis.

4. The skin conditioner according to any one of claims 1 through 3 wherein the compound represented by the formula (1) is selected from the group consisting of L-arginine, ethanolamine, 2-methoxyethylamine, O-phosphorylethanolamine, 2-ethylaminoethanol, diethanolamine, 2-dimethylaminoethanol, choline, 2-amino-2-hydroxymethyl-1,3-propanediol, noradrenalin, phenethylamine, ethylenediamine, taurine, phosphatidylethanolamine, N-(2-hydroxyethyl)acetamide, 2-(methylamino)ethanol, 2-anilinoethanol, 2-(benzylamino)ethanol, 3-amino-1-propanol, 2-amino-1-butanol, putrescine, DL-pyroglutamic acid and triethanolamine.

5. The skin conditioner according to any one of claims 1 through 3 comprising one or more kinds of ingredient selected from the group consisting of L-arginine and ions thereof and/or pharmaceutically acceptable salts thereof;
an ammonium salt and ions thereof, a compound represented by the formula (1)': 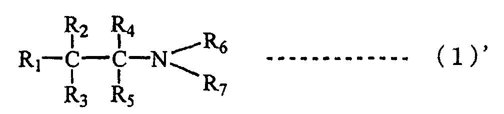 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and salts thereof and pharmaceutically acceptable salts thereof.

6. The skin conditioner according to any one of claims 1 to 3 comprising one or more kinds of ingredient selected from the group consisting of an ammonium salt and/or ions thereof;
a compound represented by the formula (1)': 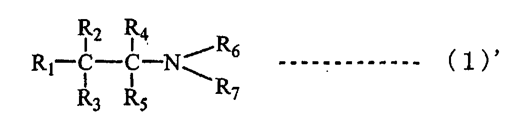 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and salts thereof and pharmaceutically acceptable salts thereof.

7. The skin conditioner according to any one of claims 1 through 3 comprising one or more kinds of ingredient selected from the group consisting of L-arginine and ions thereof and/or pharmaceutically acceptable salts thereof;
an ammonium salt and/or ions thereof, a compound represented by the formula (1)': 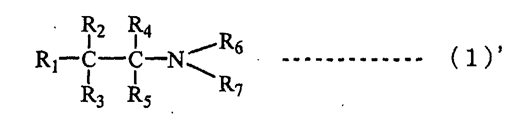 (wherein, R₁, R₂ and R₃ each independently represent a hydrogen atom, a hydroxyl group, a lower alkoxy group that may optionally have a substituent, a phosphoryloxy group, an aryl group that may optionally be substituted with a hydroxyl group, an amino group, a sulfonic acid group, a phosphatidyloxy group, a lower alkyl group that may optionally be substituted with a hydroxyl group or an amino group, a guanidino group or a lower alkyl group substituted with a guanidino group;
R₄ and R₅ each independently represent a hydrogen atom, a hydroxyl group, or a lower alkyl group or an aryl group that may optionally be substituted with a hydroxyl group or an amino group, or R₄ and R₅, together with an adjacent carbon atom, form a carbonyl group;
R₆ and R₇ each independently represent a hydrogen atom, a lower alkyl group that may optionally be substituted with a hydroxyl group, a lower alkylcarbonyl group, an aryl group or an aralkyl group, or R₆ and R₂ represent alkylene groups, which may optionally have a substituent, that together form a 5-membered ring with an adjacent atom; and
nitrogen atoms in the formula may be in a quaternary form with a lower alkyl group) and salts thereof and pharmaceutically acceptable salts thereof.

8. The skin conditioner according to any one of claims 5 through 7 wherein the compound represented by the formula (1)' is selected from the group consisting of ethanolamine, 2-methoxyethylamine, O-phosphorylethanolamine, 2-ethylaminoethanol, diethanolamine, 2-dimethylaminoethanol, choline, 2-amino-2-hydroxymethyl-1,3-propanediol, noradrenalin, phenethylamine, ethylenediamine, taurine, phosphatidylethanolamine, N- (2-hydroxyethyl)acetamide, 2-(methylamino)ethanol, 2-anilinoethanol, 2-(benzylamino)ethanol, 3-amino-1-propanol, 2-amino-1-butanol, putrescine, DL-pyroglutamic acid and triethanolamine.

9. The skin conditioner according to any one of claims 1 through 8 further containing a natural substance preparation.

10. The skin conditioner according to claim 9 wherein the natural substance preparation is a rice preparation.

11. The skin conditioner according to any one of claims 1 through 10 further containg a moisture retention agent.
